# FASCICULE DE BREVET EUROPEEN

(11) **EP 1 073 407 B1**
(45) Date de publication et mention de la délivrance du brevet: **20.04.2005**
(21) Numéro de dépôt: 99961141.1
(22) Date de dépôt: 22.12.1999
(51) Int. Cl.: A61K 7/13

(54) **PROCEDE DE TEINTURE DES CHEVEUX UTILISANT UNE AMINE CATIONIQUE ALIPHATIQUE ET UN ALDEHYDE OU CETONE OU QUINONE OU DERIVES DE LA DI-IMINO-ISOINDOLINE OU DE LA 3-AMINO-ISOINDOLONE**
VERFAHREN ZUR HAARFÄRBUNG UNTER VERWENDUNG VON EINEM KATIONISCHEN ALIPHATISCHEN AMIN UND EINEM ALDEHYD ODER EINEM KETON , EINER CHINONVERBINDUNG ODER DERIVATEN VON DIIMINO-ISOINDOLIN ODER 3-AMINO-ISOINDOLON
HAIR DYEING METHOD USING AN ALIPHATIC CATIONIC AMINE AND A ALDEHYDE OR KETONE ORQUINONE OR DI-IMINO-ISOINDOLINE OR 3-AMINO-ISO-INDOLONE DERIVATIVES

(30) Priorité: 23.12.1998 FR 9816376
(43) Date de publication de la demande: 07.02.2001
(73) Titulaire: L'OREAL, 75008 Paris (FR)
(72) Inventeur: LAGRANGE, Alain, F-77700 Coupvray (FR); ANDREAN, Hervé, F-75014 Paris (FR)
(74) Mandataire: Casalonga, Axel
(86) Numéro de dépôt international: PCT/FR1999/003247
(87) Numéro de publication internationale: WO 2000/038640

(56) Documents cités:
- EP-A- 0 502 784
- EP-A- 0 847 749
- DE-A- 4 314 317
- DE-A- 4 409 143
- GB-A- 2 181 750

## Description

La présente invention est relative à l'utilisation pour la teinture des fibres kératiniques d'au moins une molécule comportant au moins une fonction amine, au moins une chaîne hydrocarbonée aliphatique et au moins un ammonium quaternaire, et d'au moins un composé choisi parmi un aldéhyde, une cétone, une quinone, et un composé de formule (VIII) telle que définic ci-après, permettant d'obtenir, par réaction sans agent oxydant une coloration desdites fibres kératiniques ; aux compositions de teinture des fibres keratiniques comprenant au moins une molécule comportant au moins une fonction amine, au moins une chaîne hydrocarbonée aliphatique et au moins un ammonium quaternaire, et au moins un composé choisi parmi un aldéhyde, une cétone, une quinone, et un dérivé de la diiminoisoindoline ou de la 3-amino isoindolone dans un milieu approprié pour la teinture, permettant d'obtenir, sans agent oxydant, une teinture desdites fibres kératiniques ; ainsi qu'un procédé de teinture des fibres kératiniques consistant à appliquer sur lesdites fibres un composant (A) constitué d'une composition renfermant dans un milieu approprié pour la teinture, au moins une molécule comportant au moins une fonction amine, au moins une chaîne hydrocarbonée aliphatique et au moins un ammonium quaternaire, et au moins un composant (B) constitué d'une composition rcontenant dans un milieu approprié pour la teinture, au moins un composé choisi parmi un aldéhyde, une cétone, une quinone, et un dérivé de la diiminoisoindoline ou de la 3-amino isoindolone de façon à permettre le développement d'une teinture avec lesdites fibres kératiniques.

Pour la teinture des fibres kératiniques, et en particulier des fibres kératiniques humaines telles que les cheveux, il est connu d'utiliser des colorants directs ou substances colorées qui confèrent à la fibre une coloration temporaire ou semi-permanente, de faible puissance tinctoriale et qui s'élimine généralement aux lavages. Les gammes des nuances obtenues par ces procédés directs sont en général réduites. Il est également connu d'utiliser des colorants d'oxydation (bases d'oxydation et coupleurs) qui sont des composés initialement incolores ou faiblement colorés, engendrant sous l'action d'un oxydant, des composés colorés par un processus de condensation oxydative. Les colorations d'oxydation sont, comparativement aux colorations directes, permanentes, puissantes, et résistantes aux agents extérieurs (lumière, intempéries, lavages, transpiration et frottements). Néanmoins, l'utilisation de l'agent oxydant peut altérer les fibres kératiniques ct rend les procédés de mise en oeuvre des teintures oxydatives relativement complexes.

La demanderesse vient de découvrir un nouveau procédé de teinture, ne mettant pas oeuvre un processus de développement des colorants par voie oxydative, permettant d'obtenir une large gamme de nuances.

Les composés utilisés par la demanderesse sont de petites molécules qui peuvent facilement pénétrer dans la kératine. La demanderesse a constaté, de façon surprenante, que ces composés peuvent ensuite se condenser en chromophores ou colorants, molécules plus volumineuses qui restent piégées au sein de la kératine.

La demanderesse a ainsi constaté que les colorations obtenues sont résistantes aux shampooings et à la transpiration, stables à la lumière, aux intempéries et aux agents chimiques. Ces colorations sont particulièrement bien résistantes aux shampooings. En quelque sorte, la demanderesse a découvert un nouveau procédé de teinture présentant les avantages de la teinture dite d'oxydation sans en présenter les inconvénients, aucun agent oxydant n'étant utilisé.

La présente invention a donc pour objet l'utilisation pour la teinture des fibres kératiniques d'une amine cationique aliphatique et d'un composé choisi parmi un aldéhyde, une cétone, une quinone et un dérivé de la di-imino-isoindoline ou de la 3-amino-isoindolone.

Un autre objet de l'invention est relatif aux compositions de teintures comprenant ces composés.

La présente invention a aussi pour objet un procédé de teinture des fibres kératiniques consistant à appliquer sur les fibres une amine cationique aliphatique et un composé choisi parmi un aldéhyde, une cétone, une quinone et un dérivé de la diiminoisoindoline ou de la 3-amino-isoindolone, soit simultanément, sous forme d'un mélange extemporané, soit de façon successive.

Un autre objet de l'invention consiste aussi en un agent de teinture pour la mise en oeuvre du procédé de l'invention.

D'autres objets de l'invention apparaîtront à la lumière de la description et des exemples qui suivent.

L'objet principal de la présente invention est donc l'utilisation pour la teinture des fibres kératiniques, en particulier des fibres kératiniques humaines telles que les cheveux humains, d'au moins une molécule comportant au moins une fonction amine, au moins une chaîne hydrocarbonée aliphatique et au moins un ammonium quaternaire, et d'au moins un composé choisi parmi un aldéhyde, une cétone, une quinone, et un composé de formule (VIII) 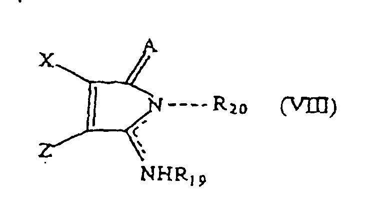 dans laquelle
R₁₉ et R₂₀, identiques ou différents, désignent un atome d'hydrogène, un groupement alkyle, mono ou polyhydroxyalkyle, alkylhydroxyalkyle, aminoalkyle, alkylaminoalkyle, (dihydroxy)alkylaminoalkyle, ou un groupement alkyle NR'R", avec R' et R" désignant alkyle ou pouvant former conjointement avec l'atome d'azote auxquels ils sont rattachés un cycle aryle ou un hétérocycle à 5 ou 6 chaînons),
A désigne un atome d'oxygène ou NH,
X et Z forment ensemble un cycle aryle ou un hétérocycle à 5 ou 6 chaînons, substitué ou non;
ou aux sels cosmétiquement acceptables de ces composés. permettant d'obtenir, par réaction sans agent oxydant une coloration desdites fibres kératiniques.

On entend par amine aliphatique cationique, au sens de l'invention, une molécule comportant au moins une fonction amine, au moins une chaîne hydrocarbonée aliphatique et au moins un ammonium quaternaire.

L'amine cationique aliphatique est choisie parmi les composés de formule (I) suivante et leurs sels cosmétiquement acceptables : 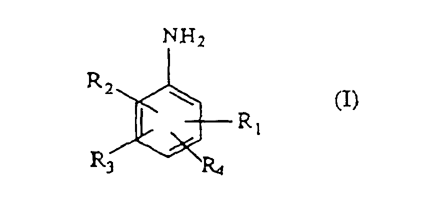 dans laquelle :
- R₁, R₂, R₃, R₄, identiques ou différents, représentent un atome d'hydrogène ; un atome d'halogène un groupement -NH₂, un groupement -OH, ; un groupement Z ; un groupe -COZ : un groupe - COOZ ; un radical alkyle carbonyle ; un radical aminoalkyle carbonyle ; un radical N-alkylaminoalkyle carbonyle ; un radical N,N-dialkylaminoalkyle carbonyle ; un radical aminoalkyle carbonylalkyle ; un radical N-alkylaminoalkyle carbonylalkyle ; un radical N,N-dialkylaminoalkyle carbonylalkyle ; un radical carboxy ; un radical alkylcarboxy ; un radical alkylsulfonyle ; un radical aminosulfonyle ; un radical N-alkylaminosulfonyle ; un radical N,N-dialkylaminosulfonyle ; un radical aminosulfonalkyle ; un radical N-alkyl aminosulfonylalkyle ; un radical N,N-dialkyl aminosulfonylalkyle ; un radical carbamyle ; un radical N-alkyl carbamyle ; un radical N,N-dialkylcarbamyle ; un radical carbamylalkyle ; un radical N-alkyl carbamylalkyle ; un radical N,N-dialkyl carbamylalkyle ; un radical alkyle, monohydroxyalkyle, polyhydroxyalkyle, alcoxyalkyle, trifluoroalkyle, un radical cyano ; un groupement ORᵢ, SRᵢ, ORᵢZ ou SRᵢZ ou un groupe amino protégé par un radical alkylcarboxy, trifluoroalkylcarbonyle, aminoalkylcarbonyle, carbonyle, N-alkylaminoalkylcarbonyle, N,N-dialkylaminoalkyl-carbonyle, alkylcarboxy, carbamyle, N-alkylcarbamyle, N,N-dialkylcarbamyle, alkylsulfonyle, aminosulfonyle, N-alkylaminosulfonyle, N,N-dialkylaminosulfonyle, thiocarbamyle, formyle, un groupe -COZ ou un groupe -COOZ ;
- Rᵢ désigne un radical alkyle, monohydroxyalkyle, polyhydroxyalkyle, un groupement Z, un radical alcoxyalkyle ; un radical aryle ; un radical benzyle, un radical carboxyalkyle, un radical alkylcarboxyalkyle, un radical cyanoalkyle, un radical carbamylalkyle, un radical N-alkylcarbamylalkyle ; un radical N,N-dialkylcarbamylakyle ; un radical trifluoroalkyle ; un radical aminosulfonylalkyle ; un radical N-alkylaminosulfonylalkyle ; un radical N,N-dialkylaminosulfonyl-alkyle ; un radical alkylsulfinylalkyle ; un radical alkylsulfonyl-alkyle ; un radical alkylcarbonylalkyle ; un radical aminoalkyle ; un radical aminoalkyle dont l'amine est substituée par un ou deux radicaux identiques ou différents choisis parmi les radicaux alkyle, monohydroxyalkyle ; polyhydroxyalkyle, alkylcarbonyle, formyle, trifluoroalkylcarbonyle, alkylcarboxy, carbamyle, N-alkylcarbamyle, N,N-dialkylcarbamyle, thiocarbamyle, alkyl-sulfonyle et parmi les groupes Z, -COZ, ou -COOZ ;
   Z représentant un groupement de formule (II) suivante : 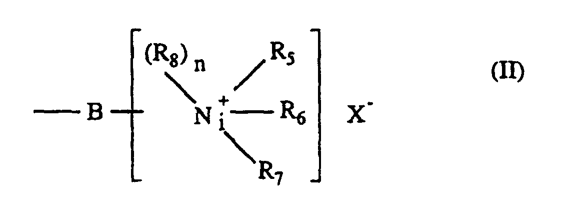 dans laquelle :
- B représente une chaîne alkyle, linéaire ou ramifiée, pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆ ;
- R₅, R₆ et R₇, identiques ou différents, représentent un radical alkyle, un radical monohydroxyalkyle, un radical polyhydroxyalkyle, un radical alcoxyalkyle, un radical cyanoalkyle, un radical aryle, un radical benzyle, un radical carbamylalkyle, un radical trialkylsilane alkyle ou un radical aminoalkyle dont l'amine est protégée par un radical alkylcarbonyle, carbamyle, ou alkylsulfonyle; deux des radicaux R₅, R₆ et R₇ peuvent également former ensemble, avec l'atome d'azote auquel ils sont rattachés, un cycle à 5 ou 6 chaînons pouvant contenir un ou plusieurs hétéroatomes, ledit cycle pouvant être ou non substitué,
   l'un des radicaux R₅, R₆ et R₇ peut également représenter un bras de liaison B' d'un second radical Z, B' ayant la même signification que celle indiquée ci-dessus pour le radical B ;
- X⁻ représente un anion monovalent ou divalent, et représente de préférence un atome d'halogène tel que le chlore, le brome, le fluor ou l'iode, un hydroxyde, un hydrogènesulfate, ou un alkylsulfate tel que par exemple un méthylsulfate ou un éthylsulfate ;
- R₈ représente un radical alkyle, monohydroxyalkyle, polyhydroxyalkyle, un radical aryle ; un radical benzyle ; un radical aminoalkyle, un radical aminoalkyle dont l'amine est protégée par un radical akylcarbonyle, carbamyle ou alkylsulfonyle; un radical carboxyalkyle ; un radical cyanoalkyle ; un radical carbamylalkyle ; un radical trifluoroalkyle ; un radical trialkylsilane alkyle ; un radical sulfonamidoalkyle ; un radical alkylcarboxyalkyle ; un radical alkylsulfinylalkyle ; un radical alkylsofonylalkyle ; un radical alkylcétoalkyle ; un radical N-alkylcarbamylalkyle ; un radical N-alkylsulfonamidoalkyle ;
- n est un nombre entier égal à 0 ou 1, étant entendu que :
   quand n = 0, le bras de liaison B est rattaché à l'atome d'azote portant les radicaux R₅ à R₇ ;
   quand n = 1, alors deux des radicaux R₅ à R₇ forment conjointement avec l'atome d'azote auquel ils sont rattachés un cycle saturé à 5 ou 6 chaînons pouvant contenir un ou plusieurs hétéroatomes, ledit cycle pouvant être substitué ou non substitué, et le bras de liaison B est porté par un atome de carbone dudit cycle saturé en dehors de l'atome d'azote Nᵢ ; et
- le composé (I) défini ci-dessus présente au moins un groupement Z.

Parmi les composés de formule (I), on peut notamment citer
- le chlorure de [2-(2,4-diamino-phénoxy)-éthyl]-diéthylméthyl-ammonium;
- le chlorure de [3-(2,4-diamino-phénoxy)-propyl]-triéthylammonium;
- le chlorure de [2-(2,4-dihydroxy-phényl)-2-oxo-éthyl]-triéthyl-ammonium;
- le chlorure de [2-(4-amino-2-hydroxy-phénoxy)-éthyl]-diéthyl-méthyl-ammonium;
- le bromure de triéthyl-[2-(3-hydroxy-4-méthylphénylamino)-éthyl]-ammonium;
- le chlorure de triéthyl-[2-(3-hydroxy-2,4-diméthylphénylcarbamoyloxy)-éthyl]-ammonium;
- le bromure de [2-(4-chloro-5-hydroxy-phénylamino)-éthyl]-triéthyl-ammonium;
- le chlorure de [2-(2,4-diamino-phénoxy)-éthyl]-diéthylméthyl-ammonium;
- le chlorure de [2(2,4-diamino-phényl)-éthyl]-triéthylammonium;
- le chlorure de triéthyl-[(3-hydroxy-4-méthylphénylcarbamoyl)-méthyl-ammonium;
- le iodure de [2-[4-(diméthylamino)-salicylamido]-éthyl]-diéthyl-méthyl ammonium;
- le bromure d'éthyl-(2-hydroxyéthyl)-diméthyl-ammonium 4-(méthylamino)-salicylate;
- le iodure de 3-[(4-amino-2-hydroxybenzoyl)-oxy]-N-éthyl-N,N-diméthyl-1-propyl ammonium;
- le iodure de 3-[(4-amino-2-hydroxybenzoyl)-oxy]-N,N,N-triméthyl-1-propyl ammonium;
- le bromure de triéthyl-(2-hydroxyéthyl)-ammonium 4-aminosalicylate;
- le iodure de 2-[(4-amino-2-hydroxybenzoyl)-oxy]-N,N-diéthyl-N-méthyl-éthyl ammonium;
- le iodure de 2-[(4-amino-2-hydroxybenzoyl)-oxy]-N-éthyl-N,N-diméthyl-éthyl ammonium;
- le bromure d'éthyl-(2-hydroxyéthyl)-diméthyl-ammonium 4-aminosalicylate;
- le iodure de 2-[(4-amino-2-hydroxybenzoyl)-oxy]-N,N,N-triméthyl-éthyl ammonium;
- le monochlorure de {2-[2-amino-phénylamino]-éthyl}triméthyl-ammonium, monohydrate;
- le monochlorure de [2-(2-amino-5-chloro-phénylamino)-éthyl]-triméthyl-ammonium;
- le monochlorure de [2-(2-amino-6-chloro-phénylamino)-éthyl]-triméthyl-ammonium;
- le monochlorure de [2-(2-amino-4-chloro-phénylamino)-éthyl]-triméthyl-ammonium;
- le monochlorure de {2-[2-amino-4-chloro-5-(2-hydroxyéthoxy)-phénylamino]- éthyl}-triméthyl-ammonium;
- le monochlorure de [2-(2-amino-5-méthoxy-phénylamino)-éthyl]-triméthyl-ammonium;
- le monobromure de [2-(2-amino-phénylamino)-éthyl]-(2-hydroxyethyl)-diméthyl-ammonium;
- le monochlorure de [3-(2-amino-phénylamino)-propyl]-diéthyl-méthyl-ammonium;
- le monochlorure de [2-(2-amino-4-méthyl-phénylamino)-éthyl]-triméthyl-ammonium;
- le chlorure de [2-(2,5-diamino-phénoxy)-éthyl]-diéthylméthyl-ammonium monohydrate;
- le dichlorure de N,N-bis-(triméthylammonium-propyl)-4-amino-aniline;
- le chlorure de [4-(4-amino-phénylamino)-pentyl]-diéthylméthyl-ammonium;
- le chlorure de [4-(4-amino-phénylamino)-pentyl]-diéthyl-(2-hydroxyéthyl)-ammonium;
- le chlorure de [2-(4-amino-phénylamino)-éthyl]-diéthylméthyl-ammonium;
- le chlorure de {2-[(4-aminophényl)-méthyl-amino]-éthyl}triméthyl-ammonium;
et leurs sels d'addition avec un acide.

Préférentiellement on utilise les composés (I) choisis parmi :
- le chlorure de [3-(4-amino-phénylamino)-propyl]-triméthylammonium;
- le chlorure de [2-(4-amino-phénylamino)-propyl]-triméthylammonium;
- le chlorure de [4-(4-amino-2-méthyl-phénylamino)-pentyl]-diéthyl-méthyl-ammonium;
- le chlorure de [4-(4-amino-3-méthyl-phénylamino)-pentyl]-diéthyl-méthyl-ammonium;
- le dibromure de N1,N4-bis-[3-N-méthyl-N-(4'-aminoaniline)-éthyl]-1,1,4,4-tétrainéthyl-diammonium 1-3-propane, dibromhydrate, monohydrate
- le dibromure de N1,N3-bis-[3-N(4'-amino-aniline)-propyl]-1,1,3,3-tétraméthyl-diammonium 1-3-propane, monohydrate;
- le dibromure de 1,3-bis-{[2-(4-amino-aniline)-propyl]-1,1,3,3-tétraméthyl-diammonium-propane;
- le dichlorure de 1,3-bis-{[4-(4-amino-aniline)-pentyl]-1,1,3,3-tétraméthyl-diammonium-propane;
- le monochlorure de [4-(4-amino-phénylamino)-pentyl]-(5-amino-2-hydroxy-benzyl)-diéthyl-ammonium;
- le monochlorure de [2-(4-amino-phénylamino)-propyl]-(5-amino-2-hydroxy-benzyl)-diméthyl-ammonium;
- le dibromure de N1,N3-bis-[3-N-(2'-amino-aniline)-propyl]-1,1,3,3-tétraméthyl-diammonium 1-3-propane,
- le dibromure de 1,3-bis-{[2-(2,4-diamino-phénoxy)-éthyl]-diéthyl-ammonium}-propane ;
et leurs sels d'addition avec un acide.

L'aldéhyde peut correspondre à la formule (III) suivante: 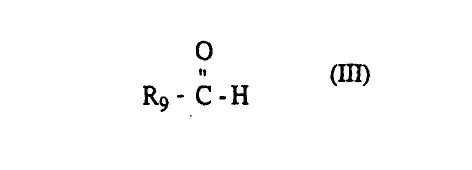 dans laquelle :
R₉ désigne un groupement de formule (III A) suivante: 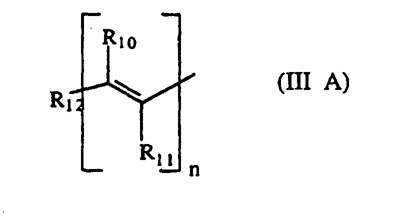 dans laquelle
R₁₀ et R₁₁, identiques ou différents, désignent un atome d'hydrogène, un groupement alkyle, mono ou polyhydroxyalkyle, alkylhydroxyalkyle, alcoxy, -CF₃ ou -OCF₃,
R₁₀ et R₁₁ peuvent également former conjointement avec les atomes auxquels ils sont rattachés un cycle aryle ou un hétérocyclique à 5 ou 6 chaînons, lesdits cycles pouvant être substitués ou non;
n désigne un nombre entier de 0 à 3,
R₁₂ désigne les substituants désignés par R₁₀, un groupement aryle, alkylaryle substitué ou non, un groupe hétérocyclique à 5 ou 6 chaînons substitué ou non,
ou aux sels cosmétiquement acceptables de ces composés.

La cétone peut être choisie parmi les cétones de formules (IV) ou (V) suivantes : 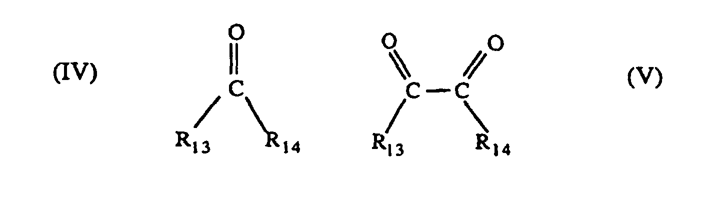 dans lesquelles :
R₁₃ désigne les substituants désignés par R₉,
R₁₄ désigne un groupement alkyle, mono ou polyhydroxyalkyle, alkylhydroxyalkyle, un groupement aryle, alkylaryle, un hétérocyclique à 5 ou 6 chaînons substitué ou non,
R₁₃ et R₁₄ peuvent également former conjointement avec les atomes auxquels ils sont rattachés un cycle aryle à 5 ou 6 chaînons, ou un hétérocyclique comprenant des hétéroatomes tels que N ou S, ledit cycle pouvant lui-même être rattaché à un cycle aryle à 5 ou 6 chaînons ou à un hétérocycle comprenant des hétéroatomes tels que N ou S, lesdits cycles pouvant être substitués ou non,
ou aux sels cosmétiquement acceptables de ces composés.

La quinone peut répondre aux formules (VI) et (VII) suivantes: 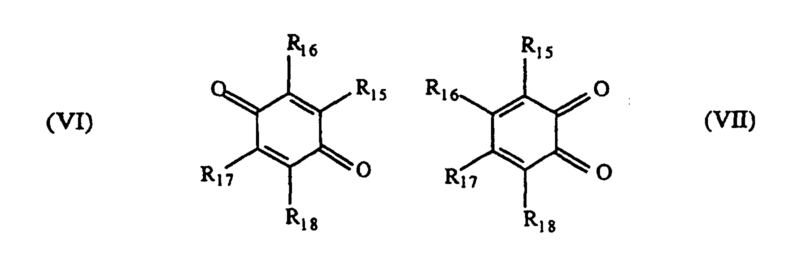 dans lesquelles :
R₁₅ désigne un atome d'hydrogène, d'halogène, un groupement sulfonique ou alcoxy,
R₁₆, R₁₇ et R₁₈, identiques ou différents désignent un atome d'hydrogène, d'halogène, un groupement hydroxy, alkyle, mono ou polyhydroxyalkyle, alkylhydroxyalkyle, alkylsulfonyle, carboxyalkyle, aminoalkyle, alkylaminoalkyle, (di-hydroxy)alkylaminoalkyle, ou alkyle-NR'R" (avec R' et R" désignant alkyle ou pouvant former ensemble avec l'atome d'azote auxquels ils sont rattachés un cycle aryle ou un hétérocycle à 5 ou 6 chaînons), un groupement aryle, un groupe amino pouvant être substitué par un alkyle ou un hydroxyalkyle,
R₁₅ et ₁₆, R₁₆ et R₁₇ ou R₁₇ et R₁₈ peuvent former conjointement avec les atomes auxquels ils sont rattachés un cycle aryle ou un hétérocycle à 5 ou 6 chaînons, substitué ou non;
ou aux sels cosmétiquement acceptables de ces composés.

Les dérivés de la di-imino-isoindoline ou de la 3-aminoisoindolone peuvent être ceux correspondant à la formule (VIII) suivante: 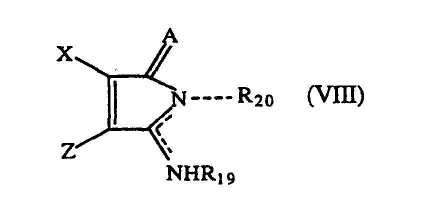 dans laquelle :
R₁₉ et R₂₀, identiques ou différents, désignent un atome d'hydrogène, un groupement alkyle, mono ou polyhydroxyalkyle, alkylhydroxyalkyle, aminoalkyle, alkylaminoalkyle, (dihydroxy)alkylaminoalkyle, ou un groupement alkyle NR'R", avec R' et
R" désignant alkyle ou pouvant former conjointement avec l'atome d'azote auxquels ils sont rattachés un cycle aryle ou un hétérocycle à 5 ou 6 chaînons),
A désigne un atome d'oxygène ou NH,
X et Z forment ensemble un cycle aryle ou un hétérocycle à 5 ou 6 chaînons, substitué ou non;
ou aux sels cosmétiquement acceptables de ces composés.

Parmi les composés préférés de formule (III), on peut notamment citer le benzaldéhyde, les 2,3,4,monohydroxybenzaldéhydes, les 2,3,4,monométhoxy-benzaldéhydes, les 2,3,4,monométhyl-benzaldéhydes, les (2,3), (2,4), (2,5), (2,6), (3,5)-dihydroxy benzaldéhydes, les (2,3), (2,4), (2,5), (2,6), (3,5)-diméthoxy benzaldéhydes, la vaniline, l'isovaniline, le syringaldéhyde, les ortho, iso, téré-phthaldéhyde, les (2,3), (2,4), (2,5), (2,6), (3,5)-diméthylbenzaldéhydes, le 4-isopropyl-benzaldéhyde, 4-diméthylaminobenzaldéhyde, 4-diéthylaminol-benzaldéhyde, le pipéronal, les (2,6), (3,5)-diméthyl-4-hydroxy-benzaldéhyde, les 2,3,4-mononitrobenzaldéhydes, le 2-hydroxy-3-méthoxy-benzaldéhyde, le 2-hydroxy-4-méthoxy-benzaldéhyde, le 2-hydroxy-5-méthoxy-benzaldéhyde, le 2-hydroxy-6-méthoxybenzaldéhyde, le 4-méthylthio-benzaldéhyde, les (2,3,4), (2,4,6), (3,4,5), (2,4,5)-trihydroxy-benzaldéhydes, les méthyles 2, 3 et 4-formylbenzoates, les 2,3,4-mono(2-hydroxyethoxy)-benzaldéhydes, le 4-nitro-3-hydroxy-benzaldéhyde, le 3-nitro-4-hydroxy-benzaldéhyde, le 2-nitro-4-hydroxy-benzaldéhyde, le 3-nitro-2-hydroxy-benzaldéhyde, les 2,3,4-monotrifluoro-benzaldéhydes, le 2,3-dihydroxy-4-méthoxy-benzaldéhyde, le 3,4-dihydroxy-5-méthoxybenzaldéhyde, le 3,5-dihydroxy-4-méthoxy-benzaldéhyde, le 3-méthoxy-2-nitrobenzaldéhyde, le 4-méthoxy-2-nitrobenzaldéhyde, le 2-méthoxy-3-nitrobenzaldéhyde, le 4-méthoxy-3-nitrobenzaldéhyde, les (2,3,4), (2,4,6), (3,4,5), (2,4,5)-triméthoxy-benzaldéhydes, la 5-nitrovaniline, les (2,4), (2,6)-dinitrobenzaldéhydes, le pentaméthylbenzaldéhyde, le 4-méthylsulfonyl-benzaldéhyde, les acides 2,3,4-monoformylphénoxyacétiques, le 4-diéthylamino-salicylaldéhyde, le 4(3-diméthylaminopropoxy)-benzaldéhyde, le 2,3-dihydrobenzo (b)furan-5-carboxaldéhyde, le 1 et le 2 naphthaldéhyde, le 6 et 5 carboxaldéhyde-1,4-benzodioxane, les 2,4-monohydroxy-1-naphtaldéhydes, le 1-monohydroxy-2-naphtaldéhyde, le 1(4-formylphényl)-imidazole, le 4-pyrrolidinol-benzaldéhyde, les 2,4 monométhoxy-1-naphthaldéhydes, le 2,3-diméthyl-chroman-6-carboxaldéhyde, le 2,3,6,7-tétrahydro-1H,5H-pyrido(3,2,1-IJ) Quinoline-9-carbaldéhyde, le 4 diméthylamino-1-naphthaldéhyde, le 9-anthraldéhyde, le 3-nitro-4-pyrrolidino-benzaldéhyde, le 3-nitro-4-pipéridino-benzaldéhyde, le 3-nitro-4-morpholino-benzaldéhyde, les pyridines 2,3,4-monocarboxaldéhydes, le 2,6-pyridinodicarboxaldéhyde, le 5-formyl-6-méthyluracil, le pyridoxale, les quinoléïnes - 2,3,4-monocarboxaldéhydes, le 8-hydroxy-quinoléïne-2-carboxaldéhyde, les 2 et 3-furaldéhydes, les 2 et 3-thiénylcarboxaldéhydes, les 2 et 3-imidazo-carboxaldéhydes, le 2-pyrrolcarboxaldéhyde, le 5-nitro-2-furaldéhyde, le 5-(diméthylamino)-2-furaldéhyde, les 2,5 et 2,3-thiophène-dicarboxaldéhydes, le pyrazol-3-carbaldéhyde, le 5-nitro-2-thiophène-carboxaldéhyde, le 5-nitro-3-thiophènecarboxaldéhyde, l'indole-3-carboxaldéhyde, le N-méthylindole-3-carboxaldéhyde, le 2-méthyl-indole-3-carboxaldéhyde, les 4,5,6,7-monométhyl-indole-carboxaldéhyde et l'acide 5-formyl-2-furansulfonique.

Les cétones de formules (IV) et (V) peuvent être choisies parmi la 2,3 indolinedione,la 2,3-butanedione, la 2,3-pentanedione, la (2,3), (3,4)-hexanedione, la 1-phényl-1,2-propanedione, le benzyl, le furil, le 2,2'-pyridil, le nitro-benzyl, l'anisil, le 3,3'-diméthoxybenzyl,le 4,4'-bis(diméthylamino)benzyl, la camphoroquinone, le cyclohexane-1,2-dione, l'isatine, la N-méthylisatine, la 4,5,6,7-monométhyl-isatine, la (4,5),(4,7),(5,7),(6,7)-diméthyl-isatine, la N-éthyl-isatine, la N-hydroxyméthyl-isatine, la 5,6,7 monométhoxy-isatine, la 4,5,6,7 monochloro-isatine, la 4,5,6,7 monobromo-isatine, la N-isopropyl-isatine, la N-butyl-isatine, la N-propyl-isatine, la 5-nitro-isatine, l'acide 5-sulfonique-isatine, la 2,4,5-trihydroxypyrimidine, l'alloxane, la 1,3-diméthyl-hexahydro-2,4,5,6-pyrimidinetetraone, la ninhydrine, la chinisatine, le 1,3-indenedione, l'acide squarique, l'acide croconique, la 3,4-diméthoxy-3-cyclobutène-1,2-dione, la 3,4-éthoxy-3-cyclobutène-1,2-dione, la 3,4-isopropoxy-3-cyclobutène-1,2-dione, la 3,4-di-N-butoxy-3-cyclobutene-1,2-dione, l'acide rhodizonique. l'oxindole, la N-méthyl-2-indolinone, la N-méthyl-nitro-2-indolinone, le 6-méthoxyoxindole, le 5,6-diméthoxyoxindole et les 5 et 6-monochlorooxindole.

Les quinones préférées de formules (VI) et (VII) sont, entre autres, la 1,4 naphtoquinone, la spinulosine, l'atromentine, l'aurentioglyocladine, la 2,5-dihydroxy-6-méthylbenzoquinone, la 2-hydroxy-3-méthyl-6méthoxylbenzoquinone, la 2,5-dihydroxy-3,6-diphénylbenzoquinone, la 2,3-diméthyl-5-hydroxy 6-méthoxybenzoquinone, la 2,5-dihydroxy 6-isopropyl-benzoquinone, la lawsone, la juglone, la fafioline, la naphtazarine, la naphtopurpurine, le lapachol, la plumbagine, la chloroplumbagine, la drosérone, la shikonine, la 2-hydroxy-3-méthyl-1,4-naphtoquinone, la 3,5-dihydroxy-1,4-naphtoquinone, la 2,5-dihydroxy-1,4-naphtoquinone, la 2-méthoxy-5-hydroxy-1,4-naphtoquinone, la 3-méthoxy-5-hydroxy-1,4-naphtoquinone, la (1,4),(1,2)naphtoquinone, la 4,5-diméthoxy-1,2-benzoquinone, la phenanthrènequinone et l'acide 4-sulfonique(1,2)naphtoquinone.

Les dérivés de formule (VIII) sont notamment représentés par la 3-imino-3H-isoindol-ylamine, la 3-imino-4-méthyl-3H-isoindol-1-ylamine, la 3-imino-4-terbutyl-3H-isoindol-1-ylamine, la 3-imino-7-nitro-3H-isoindol-1-ylamine, la 3-amino-1-imino-1H-isoindol-4-ol, la 3-imino-7-isopropoxy-3H-isoindol-1-ylamine, la 3-imino-7-(2,2,2-trifluoroéthoxy)-3H-isoindol-1-ylamine, la 3-imino-7-éthoxy-3H-isoindol-1-ylamine, la 3-imino-7-butoxy-3H-isoindol-1-ylamine, l'acide 3-amino-1-imino-1H-isoindole-4-sulfonique, la 3-imino-7-chloro-3H-isoindol-1-ylamine, la 3-imino-5-méthyl-3H-isoindol-1-yiamine, la 3-imino-5-éthyl-3H-isoindol-1-ylamine, la 3-imino-5-terbutyl-3H-isoindol-1-ylamine, la 3-imino-5-amino-3H-isoindol-1-ylamine, la N-(1-amino-3-imino-3H-isoindol-5-yl)-acétamide, la 3-imino-5-nitro-3H-isoindol-1-ylamine, la 3-imino-5-fluoro-3H-isoindol-1-ylamine, la 3-imino-5-chloro-3H-isoindol-1-ylamine, la 3-imino-5-méthylsulfanyl-3H-isoindol-1-ylamine, la 3-imino-5-méthoxy-3H-isoindol-1-ylamine, la 3-imino-5-éthoxy-3H-isoindol-1-ylamine, la 3-imino-5-propoxy-3H-isoindol-1-ylamine, la 3-imino-5-isopropoxy-3H-isoindol-1-ylamine, la 3-imino-5-butoxy-3H-isoindol-1-ylamine, la 3-imino-5-isobutoxy-3H-isoindol-1-ylamine, la 3-imino-5-terbutoxy-3H-isoindol-1-ylamine, la 3-imino-5-(2,2,2-trifluorométhyl)-3H-isoindol-1-ylamine, la 3-imino-5-(2,2,2-trifluoroéthoxy)-3H-isoindol-1-ylamine, la 3-imino-5-méthanesulfonyl-3H-isoindol-1-ylamine, la 3-imino-5,6-diméthyl-3H-isoindol-1-ylamine, la 3-imino-5,6-diéthyl-3H-isoindol-1-ylamine, la 3-imino-5,6-diméthoxy-3H-isoindol-1-ylamine, la 3-imino-5,6-diéthoxy-3H-isoindol-1-ylamine, la 3-imino-5,6-dibutoxy-3H-isoindol-1-ylamine, la 3-imino-5,6-bis-trifluorométhyl-3H-isoindol-1-ylamine, la 3-imino-5,6-dichloro-3H-isoindol-1-ylamine, la 5,6-bis-éthoxyméthyl-3-imino-3H-isoindol-1-ylamine, la 3-amino-1-imino-1H-isoindol-4,7-diol, la 4,7-dichloro-3-imino-3H-isoindol-1-ylamine, la 4,5,7-trichloro-3-imino-N6,N6-diméthyl-3H-isoindol-1,6-diamine, la 4,5,6,7-tétrachloro-3-imino-3H-isoindol-1-ylamine, la 4,5,6,7-tétrafluoro-3-imino-3H-isoindol-1-ylamine, la 3-butylimino-3H-isoindol-1-ylamine, la 2-(3-amino-isoindol-1-ylidèneamino)-éthanol, la 3-(3-amino-isoindol-1-ylidèneamino)-3-méthyl-pentane-1,5-diol, la N-(3-amino-isoindol-1-ylidène)-guanidine, la 7-imino-7H-pyrrolo[3,4-b]pyridin-5-ylamine, la 7-imino-7H-pyrrolo[3,4-b]pyrazin-5-ylamine, la 7-imino-2,3-diméthyl-7H-pyrrolo[3,4-b]pyrazin-5-ylamine, la 7-imino-7H-[1,4]dithiino[2,3-c] pyrrol-5-ylamine, la 7-imino-2,3-diméthyl-7H-[1,4]dithiino[2,3-c] pyrrol-5-ylamine, la 7-imino-2,3-dihydro-7H-[1,4]dithiino[2,3-c]pyrrol-5-ylamine, la 7-imino-2-méthyl-2,3-dihydro-7H-[1,4]dithiino [2,3-c]pyrrol-5-ylamine, la 3-amino-isoindol-1-one, la 3-amino-7-méthyl-isoindol-1-one, la 3-amino-7-hydroxyméthyl-isoindol-1-one, la 3-amino-7-chloro-isoindol-1-one, la 3-amino-4-chloro-isoindol-1-one, l'acide 3-amino-1-oxo-1H-isoindole-4-sulfonique, la 3-amino-4-nitroisoindol-1-one, la 3-amino-6-nitro-isoindol-1-one, la 3-amino-6-méthyl-isoindol-1-one, la 3-amino-6-chloro-isoindol-1-one, la 3-amino-6-bromo-isoindol-1-one, la 3-amino-6-méthylsulfanyl-isoindol-1-one, la 3-amino-6-méthoxy-isoindol-1-one, la 3-amino-5-chloroisoindol-1-one, la 3-amino-5-fluoro-isoindol-1-one, la 3-amino-5-méthoxy-isoindol-1-one, la 3-amino-5-nitro-isoindol-1-one, l'ester éthylique de l'acide 3-amino-1-oxo-1H-isoindole-5-carboxylique, la 3-amino-5,6-dichloro-isoindol-1-one, la 3-amino-5,6-dibromo-isoindol-1-one, la 3-amino-4,7-dichloro-isoindol-1-one, la 3-amino-4,5,7-trichloro-isoindol-1-one, la 3-amino-4,5,6,7-tétrachloro-isoindol-1-one, la 3-amino-4,5,7-trichloro-6-méthylsulfanyl-isoindol-1-one, la 3-amino-4,5,6,7-tétrabromo-isoindol-1-one, la 3-amino-4,5,6,7-tétrafluoro-isoindol-1-one, la 3-méthylamino-isoindol-1-one, la 3-éthylamino-isoindol-1-one, la 3-propylamino-isoindol-1-one, la 3-diméthylamino-isoindol-1-one, la 7-éthylamino-pyrrolo[3,4-b]pyridin-5-one, la 7-amino-pyrrolo[3,4-b]pyridin-5-one, la 3-aminopyrrolo[3,4-c]pyridin-5-one, la 3-amino-6-méthyl-pyrrolo[3,4-c]pyridin-1-one, la 5-amino-pyrrolo[3,4-b]pyridin-7-one, la 7-aminopyrrolo[3,4-b]pyrazin-5-one, la 7-amino-2-méthyl-pyrrolo[3,4-b]pyrazin-5-one, la 7-amino-2,3-diméthyl-pyrrolo[3,4-b]pyrazin-5-one, la 7-amino-2,3-dihydro-[1,4]dithiino[2,3-c]pyrrol-5-one, la 3-imino-2-méthyl-2,3-dihydro-isoindol-1-one, la 3-imino-2-éthyl-2,3-dihydro-isoindol-1-one, la 3-imino-2-propyl-2,3-dihydro-isoindol-1-one, la 2-hydroxyméthyl-3-imino-2,3-dihydro-isoindol-1-one, la 2-(2-hydroxyéthyl)-3-imino-2,3-dihydro-isoindol-1-one, l'acide 2-(1-imino-3-oxo-1,3-dihydro-isoindol-2-yl)-éthane sulfonique, l'acide 3-(1-imino-3-oxo-1,3-dihydro-isoindol-2-yl)-propionique, la 2-(3-hydroxypropyl)-3-imino-2,3-dihydro-isoindol-1-one et la 5-imino-6-méthyl-5,6-dihydro-pyrrolo[3,4-b]pyridin-7-one.

Dans le cadre de la présente invention:

Les atomes d'halogène désignent préférentiellement un atome de fluor, de chlore, de bromure ou d'iode.

Les radicaux alkyle, monohydroxyalkyle, polyhydroxyalkyles, alkylhydroxyalkyle, alkylesulfonyle, carboxyalkyle, aminoalkyle, alkylaminoalkyle, dihydroxyaminoalkyle peuvent être linéaires ou ramifiés.

Les groupements alkyle désignent notamment les groupements de 1 à 20 atomes de carbone, comme par exemple, les groupements méthyle, éthyle, propyle, isopropyle, n-propyle, butyle, n-butyle, tert-butyle, pentyle, n-pentyle, isopentyle, n-hexyle, isohexyle, heptyle, octyle, nonyle, decyle, undecyle, dodecyle et pentadecyle. Préférentiellement, les groupements alkyle désignent un groupement de 1 à 6 atomes de carbone ;
ces groupements alkyles peuvent être substitués; par exemple, par un atome d'halogène, un radical cyano ou hydroxy, et peuvent ainsi représenter les radicaux trifluorométhyle, δ-chloropropyle, β-cyanoéthyle ou β-hydroxyéthyle.

Parmi les groupements monohydroxyalkyle, on peut notamment citer les groupements hydroxyméthyle, hydroxyéthyle, hydroxypropyle et hydroxybutyle.

Parmi les radicaux polyhydroxyalkyle, on peut par exemple citer les radicaux dihydroxyéthyle, dihydroxypropyle, trihydroxypropyle et dihydroxybutyle.

Les groupements alcoxy désignent un groupement -O-R, R représentant un groupement alkyle tel que défini ci-dessus.

Les groupements alcényles désignent un radical monovalent correspondant aux carbones éthyléniques, tels que, par exemple, alkyle ou 3,3diméthylallyle.

Les groupements acétyloxy désignent un groupement -O-CO-R, R représentant un groupement alkyle tel que défini ci-dessus.

Parmi les radicaux cycloalkyle, on peut notamment citer le cyclohexyle et le cyclopentyle.

Parmi les radicaux aryle, qui peuvent être mono ou polycycliques, on peut notamment citer les groupements phényle ou naphtyle.

Parmi les hétérocycles et notamment les cycles à 5 ou 6 chaînons, qui peuvent être mono ou polycycliques et contenant un ou plusieurs hétéroatomes, on peut citer les cycles thiophène, pyrrole, imidazole, pyrazole, triazole, thiazole, furane, benzofurane, benzimidazole, benzothiazole, pyridyle, benzoxazole, quinolyle, quinazoyle, quinoxalyle, pyrrolidine, pipéridine, pipérazine ou morpholine.

Parmi les radicaux alkylaryle, on peut notamment citer le groupement benzyle, phenethyle ou naphthylméthyle.

Les groupements aminoaryle désignent les groupements NH₂-R, R représentant un radical aryle.

Dans le cadre de la présente invention, les radicaux cycloakyles, aryle et les hétérocycles peuvent être substitués ou polysubstitués par exemple par un halogène, par un alkyle ou monohydroxyalkyle en C₁-C₆, un radical polyhydroxyalkyle en C₂-C₆, un alkoxy en C₁-C₆, un groupe nitro, un groupe hydroxy, un groupe carboxylique, un groupe acétyloxy en C₁-C₄, un groupe carboxamide, un groupe sulfonamide, sulfonique, nitrile, -CF₃ ou -OCF₃, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical alkylcarbonyl en C₁-C₆, un radical thio, un radical thioalkyle en C₁-C₆, un radical alkyl(C₁-C₆)thio, un radical amino, un radical amino protégé par un radical alkyl(C₁-C₆ )carbonyle, carbamyle ou alkyl(C₁-C₆)sulfonyle.

Dans le cadre de la présente invention, les formules (I) à (VIII) ne sont pas limitées à celles spécifiquement décrites mais comprennent aussi leurs formes tautomères quand elles existent.

Au sens de la présente invention, les sels cosmétiquement acceptables des composés précités peuvent être des chlorhydrates, des sulfates, des bromhydrates ou des tartrates.

Les compositions de teinture des fibres kératiniques, en particulier des fibres kératiniques humaines telles que les cheveux, conformes à la présente invention sont essentiellement caractérisées par le fait qu'elles comprennent au moins une amine cationique aliphatique telle que définie ci-dessus et au moins un composé choisi parmi un aldhéhyde, une cétone, une quinone et un dérivé de la di-imino-isoindoline ou de la 3-amino-isoindolone tel que défini ci-dessus, dans un milieu approprié pour la teinture.

Dans une forme de réalisation préférée de l'invention, le composé choisi parmi un aldéhyde, une cétone, une quinone et un dérivé de la di-imino-isoindoline ou de la 3-amino-isoindolone est choisi parmi la 1,4-diméthylaminobenzaldéhyde et la 4-diméthylaminonaphtaldéhyde.

L'amine cationique aliphatique peut être présente dans une concentration allant de 0,01 à 10 %, et préférentiellement entre 0,05 et 5 % en poids par rapport au poids total de la composition.

Le composé choisi parmi un aldéhyde, une cétone, une quinone et un dérivé de la di-imino-isoindoline ou de la 3-amino-isoindolone peut être présent dans une concentration allant de 0,01 à 10 %et préférentiellement de 0,05 à 5 % en poids par rapport au poids total de la composition.

Le milieu approprié pour la teinture est de préférence un milieu aqueux constitué par de l'eau et/ou des solvants organiques acceptables sur le plan cosmétique, et plus particulièrement, des alcools tels que l'alcool éthylique, l'alcool isopropylique, l'alcool benzylique, et l'alcool phényléthylique, ou des glycols ou éthers de glycol tels que, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, dans des concentrations comprises entre environ 0,5 et 20% et, de préférence, entre environ 2 et 10% en poids par rapport au poids total de la composition.

On peut également ajouter à la composition selon l'invention des amides gras tels que les mono- et di-éthanolamides des acides dérivés du coprah, de l'acide laurique ou de l'acide oléïque, à des concentrations comprises entre environ 0,05 et 10% en poids.

On peut encore ajouter à la composition selon l'invention des agents tensio-actifs bien connus de l'état de la technique et de type anionique, cationique, non-ionique, amphotère, zwittérionique ou leurs mélanges, de préférence en une proportion comprise entre environ 0,1 et 50% en poids et avantageusement entre environ 1 et 20% en poids par rapport au poids total de la composition.

On peut également utiliser des agents épaississants dans une proportion allant d'environ 0,2 à 20%.

Ladite composition tinctoriale peut contenir en outre divers adjuvants usuels tels que des agents anti-oxydants, des parfums, des agents séquestrants, des agents dispersants, des agents de conditionnement du cheveu, des agents conservateurs, des agents opacifiants, ainsi que tout autre adjuvant utilisé habituellement en teinture des matières kératiniques.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut être formulée à pH acide, neutre ou alcalin, le pH pouvant varier par exemple de 2 à 11 et de préférence de 5 à 10, et pouvant être ajusté au moyen d'agents d'alcalinisation ou d'agents d'acidification ou de tampons antérieurement bien connus.

Comme agents alcalinisants, on peut citer l'ammoniaque, les carbonates alcalins, les alcanolamines, par exemple les mono- di- et tri- éthanolamines et leurs dérivés, les hydroxydes de sodium ou de potassium, et les composés de formule : 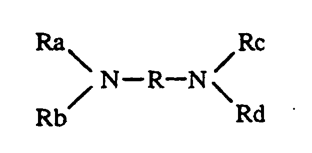 dans laquelle, R est un reste propylène éventuellement substitué par un groupement hydroxyle ou un radical alkyle en C₁-C₄; Ra, Rb, Rc et Rd, simultanément ou indépendamment l'un de l'autre représentent un atome d'hydrogène, un radical alkyle en C₁-C₄ ou hydroxyalkyle en C₁-C₄.

Les agents acidifiants sont classiquement des acides minéraux ou organiques comme par exemple les acides chlorhydriques, tartrique, citrique et phosphorique.

Parmi les tampons, on peut citer par exemple, le phosphate diacide de potassium/hydroxyde de sodium.

La composition appliquée sur les cheveux peut se présenter sous des formes diverses, telles que sous forme de liquide, de crème, de gel ou sous toute autre forme appropriée pour réaliser une teinture des fibres kératiniques. En particulier, elle peut être conditionnée sous pression en flacon aérosol en présence d'un agent propulseur et former une mousse.

Conformément à la présente invention, le procédé de teinture des fibres kératiniques, en particulier des fibres kératiniques humaines telles que les cheveux, est essentiellement caractérisé par le fait que l'on applique sur lesdites fibres un composant (A) constitué d'une composition renfermant dans un milieu approprié pour la teinture, au moins une amine cationique, comme par exemple un composé tel que ceux définis ci-dessus, et un composant (B) constitué d'une composition contenant dans un milieu approprié pour la teinture, au moins un composé choisi parmi un aldehyde, une cétone, une quinone et un dérivé de la di-imino-isoindoline ou de la 3-amino-isoindolone tel que, par exemple, un de ceux définis ci-dessus, de façon à permettre le développement d'une teinture sur lesdites fibres kératiniques.

Dans une forme de réalisation préférée du procédé de l'invention, les composants (A) et (B) sont mélangés juste avant emploi, puis la composition résultante est immédiatement appliquée sur les fibres kératiniques, et laissée agir pendant 1 à 60 minutes et préférentiellement de 1 à 30 minutes ; les fibres kératiniques étant ensuite rincées, lavées au shampooing, rincées à nouveau, puis séchées.

Un autre procédé de la présente invention consiste essentiellement à appliquer sur les fibres kératiniques le composant (A), suivi ou précédée de l'application sur lesdites fibres du composant (B), à laisser agir chaque composant pendant 1 à 60 minutes et préférentiellement de 1 à 30 minutes, à procéder éventuellement au rinçage à l'eau entre chaque application; les fibres kératiniques étant ensuite rincées, lavées au shampooing, rincées à nouveau, puis séchées.

Un objet de l'invention est aussi constitué par un agent de teinture pour les fibres kératiniques, en particulier des cheveux humains, caractérisé par le fait qu'il est constitué par les composants (A) et (B) stockés sous forme séparée, tels que définis ci-dessus.

Les composants (A) et (B) sont destinés, soit à être mélangés tous juste avant emploi, soit à être appliqués de façon successive sur les fibres à traiter.

Selon une forme de réalisation, on peut conditionner les différents composants (A) et (B) dans un dispositif à plusieurs compartiments encore appelé "kit de teinture" comportant tous les composants destinés à être appliqués pour une même teinture sur les fibres kératiniques, en particulier les fibres kératiniques humaines telles que les cheveux, en applications successives avec ou sans prémélange.

De tels dispositifs peuvent comporter un premier compartiment contenant le composant (A) renfermant l'amine cationique aliphatique et un second compartiment comportant le composant (B) renfermant le composé choisi parmi un aldéhyde, une cétone, une quinone et un dérivé de la di-imino-isoindoline ou de la 3-amino-isoindolone.

Une autre variante peut également consister à stocker le composant (A) ou le composant (B) dans un milieu solvant anhydre et à prévoir un troisième compartiment contenant un milieu aqueux approprié pour la teinture et cosmétiquement acceptable. Dans ce cas, on mélange tout juste avant l'emploi le contenu du troisième compartiment dans l'un ou l'autre ou les deux compartiments contenant les composants anhydres (A) et (B) ou alors on mélange avant emploi les trois compartiments.

Des exemples concrets illustrant l'invention vont maintenant être donnés.

### EXEMPLE 1

La composition de teinture suivante a été préparée juste avant emploi :
- 1H-indole-2,3-dione (3.10⁻³mole) 0,441 g
- chlorhydrate de chlorure de [2-(4-amino-phénylamino)-éthyl]-diéthyl-méthyl-ammonium (3.10⁻³mole) 0,99 g
- alcool éthylique 20 g
- triéthanolamine q.s.p. pH 7
- eau q.s.p. 100 g

On a appliqué à température ambiante la composition ci-dessus sur cheveux gris naturels permanentés ou non, ou sur des cheveux décolorés à raison de 5 grammes par gramme de cheveux. On procède ensuite à un rinçage à l'eau courante et à un séchage des cheveux.

Les colorations obtenues sont indiquées dans le tableau ci-dessous :

| Cheveux gris naturels | Cheveux décolorés | Cheveux gris permanentés |
|---|---|---|
| cuivre rouge | cuivre rouge | cuivre rouge |

Ces colorations sont particulièrement résistantes aux lavages aux shampooings.

### EXEMPLE 2

La composition de teinture suivante a été préparée juste avant emploi :
- 1H-indole-2,3-dione (3.10⁻³mole) 0,441 g
- chlorhydrate de chlorure de [2-(4-amino-phénylamino)-éthyl)-diéthyl-méthyl-ammonium (3.10⁻³mole) 0,99 g
- alcool éthylique 20 g
- triéthanolamine q.s.p. pH 4
- eau q.s.p. 100 g

On a appliqué à température ambiante la composition ci-dessus sur cheveux gris naturels permanentés ou non, ou sur des cheveux décolorés à raison de 5 grammes par gramme de cheveux. On procède ensuite à un rinçage à l'eau courante et à un séchage des cheveux.

Les colorations obtenues sont indiquées dans le tableau ci-dessous :

| Cheveux gris naturels | Cheveux décolorés | Cheveux gris permanentés |
|---|---|---|
| cuivré | cuivre rouge | cuivré |

Ces colorations sont tenaces en particulier vis-à-vis des shampooings.

### EXEMPLE 3

La composition de teinture suivante a été préparée juste avant emploi:
- 1H-indole-2,3-dione (3.10⁻³mole) 0,441 g
- chlorhydrate de chlorure de [2-(2,5-diaminophénoxy)-éthyl]-diéthyl-méthyl-ammonium (3.10⁻³mole) 1,09 g
- alcool éthylique 20 g
- triéthanolamine q.s.p. pH 7
- eau q.s.p. 100 g

On a appliqué à température ambiante la composition ci-dessus sur cheveux gris naturels permanentés ou non, ou sur des cheveux décolorés à raison de 5 grammes par gramme de cheveux. On procède ensuite à un rinçage à l'eau courante et à un séchage des cheveux.

Les colorations obtenues sont indiquées dans le tableau ci-dessous :

| Cheveux gris naturels | Cheveux décolorés | Cheveux gris permanentés |
|---|---|---|
| bois de rose | châtaigne clair | châtaigne clair |

Ces colorations sont particulièrement résistantes aux shampooings.

### EXEMPLE 4

La composition de teinture suivante a été préparée juste avant emploi :
- 1H-indole-2,3-dione (3.10⁻³mole) 0,441 g
- chlorhydrate de chlorure de [2-(2,5-diaminophénoxy)-éthyl]-diéthyl-méthyl-ammonium (3.10⁻³mole) 1,09 g
- alcool éthylique 20 g
- triéthanolamine q.s.p. pH 4
- eau q.s.p. 100 g

On a appliqué à température ambiante la composition ci-dessus sur cheveux gris naturels permanentés ou non, ou sur des cheveux décolorés à raison de 5 grammes par gramme de cheveux. On procède ensuite à un rinçage à l'eau courante et à un séchage des cheveux.

Les colorations obtenues sont indiquées dans le tableau ci-dessous :

| Cheveux gris naturels | Cheveux décolorés | Cheveux gris permanentés |
|---|---|---|
| beige rosé | bois de rose intense | bois de rose |

Ces colorations sont tenaces en particulier vis-à-vis des sharnpooings.

## Revendications

1. Utilisation pour la teinture des fibres kératiniques, en particulier des fibres kératiniques humaines telles que les cheveux humains, d'au moins une molécule comportant au moins une fonction amine, au moins une chaîne hydrocarbonée aliphatique et au moins un ammonium quaternaire et d'au moins un composé choisi parmi un aldéhyde, une cétone, une quinone, et un composé de formule (VIII) : 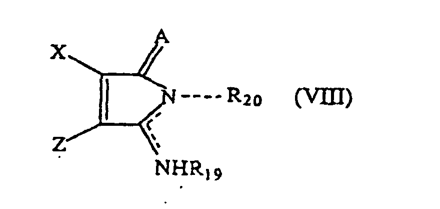 dans laquelle
R₁₉ et R₂₀, identiques ou différents, désignent un atome d'hydrogène, un groupement alkyle, mono ou polyhydroxyalkyle, alkylhydroxyalkyle, aminoalkyle, alkylaminoalkyle, (dihydroxy)alkylaminoalkyle, ou un groupement alkyle NR'R", avec R' et R" désignant alkyle ou pouvant former conjointement avec l'atome d'azote auxquels ils sont rattachés un cycle aryle ou un hétérocycle à 5 ou 6 chaînons),
A désigne un atome d'oxygène ou NH,
X et Z forment ensemble un cycle aryle ou un hétérocycle à 5 ou 6 chaînons, substitué ou non;
ou aux sels cosmétiquement acceptables de ces composés. permettant d'obtenir, par réaction sans agent oxydant une coloration desdites fibres kératiniques.

2. Utilisation selon la revendication 1, **caractérisée en ce que** la molécule comportant au moins une fonction amine, au moins une chaîne hydrocarbonée aliphatique et au moins un ammonium quaternaire est choisie parmi les composés de formule (I) suivante : 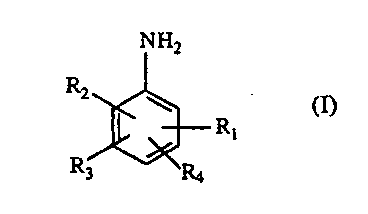 dans laquelle :
• R₁, R₂, R₃, R₄ identiques ou différents, représentent un atome d'hydrogène ; un atome d'halogène ; un groupement -NH₂, un groupement -OH, un groupement Z ; un groupe -COZ ; un groupe -COOZ ; un radical alkyle carbonyle ; un radical aminoalkyle carbonyle ; un radical N-alkylaminoalkyle carbonyle ; un radical N,N-dialkylaminoalkyle carbonyle ; un radical aminoalkyle carbonylalkyle ; un radical N-alkylaminoalkyle carbonylalkyle; un radical N,N-dialkylaminoalkyle carbonylalkyle ; un radical carboxy ; un radical alkylcarboxy; un radical alkylsulfonyle; un radical aminosulfonyle ; un radical N-alkylaminosulfonyle ; un radical N,N-dialkylaminosulfonyle ; un radical aminosulfonalkyle ; un radical N-alkyl aminosulfonylalkyle ; un radical N,N-dialkyl aminosulfonylalkyle ; un radical carbamyle ; un radical N-alkyl carbamyle ; un radical N,N-dialkylcarbamyle ; un radical carbamylalkyle ; un radical N-alkyl carbamylalkyle ; un radical N,N-dialkyl carbamylalkyle ; un radical alkyle, monohydroxyalkyle, polyhydroxyalkyle, alcoxyalkyle, trifluoroalkyle, un radical cyano ; un groupement ORᵢ ; SRᵢ, ORᵢZ ou SRᵢZ ou un groupe amino protégé par un radical alkylcarboxy, trifluoroalkylcarbonyle, aminoalkylcarbonyle, carbonyle, N-alkylaminoalkylcarbonyle, N,N-dialkylaminoalkyl-carbonyle, alkylcarboxy, carbamyle, N-alkylcarbamyle, N,N-dialkylcarbamyle, alkylsulfonyle, aminosulfonyle, N-alkylaminosulfonyle, N,N-dialkylaminosulfonyle, thiocarbamyle, formyle, un groupe -COZ ou un groupe -COOZ ;
• Rᵢ désigne un radical alkyle, monohydroxyalkyle, polyhydroxyalkyle, un groupement Z, un radical alcoxyalkyle ; un radical aryle ; un radical benzyle, un radical carboxyalkyle, un radical alkylcarboxyalkyle, un radical cyanoalkyle, un radical carbamylalkyle, un radical N-alkylcarbamylalkyle ; un radical N,N-dialkylcarbamylakyle ; un radical trifluoroalkyle ; un radical aminosulfonylalkyle ; un radical N-alkylaminosulfonylalkyle ; un radical N,N-dialkylaminosulfonyl-alkyle ; un radical alkylsulfinylalkyle ; un radical alkylsulfonyl-alkyle ; un radical alkylcarbonylalkyle ; un radical aminoalkyle ; un radical aminoalkyle dont l'amine est substituée par un ou deux radicaux identiques ou différents choisis parmi les radicaux alkyle, monohydroxyalkyle ; polyhydroxyalkyle, alkylcarbonyle, formyle, trifluoroalkylcarbonyle, alkylcarboxy, carbamyle, N-alkylcarbamyle, N,N-dialkylcarbamyle, thiocarbamyle, alkyl-sulfonyle et parmi les groupes Z, -COZ, ou -COOZ ;
Z représentant un groupement de formule (II) suivante : 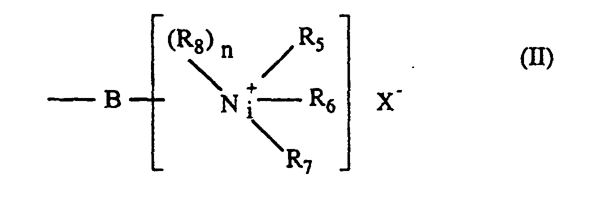 dans laquelle :
• B représente une chaîne alkyle, linéaire ou ramifiée, pouvant être interrompue par un ou plusieurs hétéroatomes tels que des atomes d'oxygène, de soufre ou d'azote, et pouvant être substituée par un ou plusieurs radicaux hydroxyle ou alcoxy en C₁-C₆ ;
• R₅, R₆ et R₇, identiques ou différents, représentent un radical alkyle, un radical monohydroxyalkyle, un radical polyhydroxyalkyle, un radical alcoxyalkyle, un radical cyanoalkyle, un radical aryle, un radical benzyle, un radical carbamylalkyle, un radical trialkylsilane alkyle ou un radical aminoalkyle dont l'amine est protégée par un radical alkylcarbonyle, carbamyle, ou alkylsulfonyle; deux des radicaux R₅, R₆ et R₇ peuvent également former ensemble, avec l'atome d'azote auquel ils sont rattachés, un cycle à 5 ou 6 chaînons pouvant contenir un ou plusieurs hétéroatomes, ledit cycle pouvant être ou non substitué,
l'un des radicaux R₅, R₆ et R₇ peut également représenter un bras de liaison B' d'un second radical Z, B' ayant la même signification que celle indiquée ci-dessus pour le radical B ;
• X⁻ représente un anion monovalent ou divalent, et représente de préférence un atome d'halogène tel que le chlore, le brome, le fluor ou l'iode, un hydroxyde, un hydrogènesulfate, ou un alkylsulfate tel que par exemple un méthylsulfate ou un éthylsulfate ;
• R₈ représente un radical alkyle, monohydroxyalkyle, polyhydroxyalkyle, un radical aryle ; un radical benzyle ; un radical aminoalkyle, un radical aminoalkyle dont l'amine est protégée par un radical akylcarbonyle, carbamyle ou alkylsulfonyle; un radical carboxyalkyle ; un radical cyanoalkyle ; un radical carbamylalkyle ; un radical trifluoroalkyle; un radical trialkylsilane alkyle ; un radical sulfonamidoalkyle ; un radical alkylcarboxyalkyle ; un radical alkylsulfinylalkyle ; un radical alkylsofonylalkyle ; un radical alkylcétoalkyle ; un radical N-alkylcarbamylalkyle ; un radical N-alkylsulfonamidoalkyle ;
• n est un nombre entier égal à 0 ou 1, étant entendu que :
quand n = 0, le bras de liaison B est rattaché à l'atome d'azote portant les radicaux R₅ à R₇ ;
quand n = 1, alors deux des radicaux R₅ à R₇ forment conjointement avec l'atome d'azote auquel ils sont rattachés un cycle saturé à 5 ou 6 chaînons pouvant contenir un ou plusieurs hétéroatomes, ledit cycle pouvant être substitué ou non substitué, et le bras de liaison B est porté par un atome de carbone dudit cycle saturé en dehors de l'atome d'azote Nᵢ ; et
• le composé (I) défini ci-dessus présente au moins un groupement Z ; et les sels cosmétiquement acceptables de ces composés.

3. Utilisation selon la revendication 1 ou 2, **caractérisée en ce que** l'aldéhyde correspond à la formule (III) suivante: 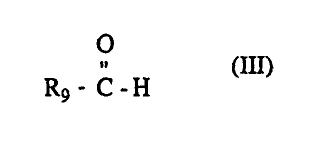 dans laquelle:
R₉ désigne un groupement de formule (III A) suivante: 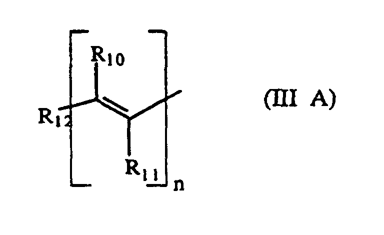 dans laquelle
R₁₀ et R₁₁, identiques ou différents, désignent un atome d'hydrogène, un groupement alkyle, mono ou polyhydroxyalkyle, alkylhydroxyalkyle, alcoxy, -CF₃ ou -OCF₃,
R₁₀ et R₁₁ peuvent également former conjointement avec les atomes auxquels ils sont rattachés un cycle aryle ou un hétérocyclique à 5 ou 6 chaînons, lesdits cycles pouvant être substitués ou non;
n désigne un nombre entier de 0 à 3,
R₁₂ désigne les substituants désignés par R₁₀, un groupement aryle, alkylaryle substitué ou non, un groupe hétérocyclique à 5 ou 6 chaînons substitué ou non,
ou aux sels cosmétiquement acceptables de ces composés.

4. Utilisation selon l'une quelconque des revendications 1 à 3, **caractérisée en ce que** la cétone correspond aux formules (IV) ou (V) suivantes : 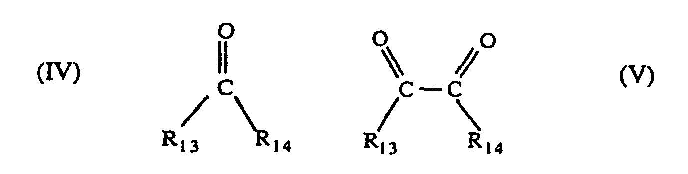 dans lesquelles :
R₁₃ désigne les substituants désignés par R₉,
R₁₄ désigne un groupement alkyle, mono ou polyhydroxyalkyle, alkylhydroxyalkyle, un groupement aryle, alkylaryle, un hétérocyclique à 5 ou 6 chaînons substitué ou non,
R₁₃ et R₁₄ peuvent également former conjointement avec les atomes auxquels ils sont rattachés un cycle aryle à 5 ou 6 chaînons, ou un hétérocyclique comprenant des hétéroatomes tels que N ou S, ledit cycle pouvant lui-même être rattaché à un cycle aryle à 5 ou 6 chaînons ou à un hétérocycle comprenant des hétéroatomes tels que N ou S, lesdits cycles pouvant être substitués ou non,
ou aux sels cosmétiquement acceptables de ces composés.

5. Utilisation selon l'une quelconque des revendications 1 à 4, **caractérisée en ce que** la quinone correspond aux formules (VI) et (VII) suivantes : 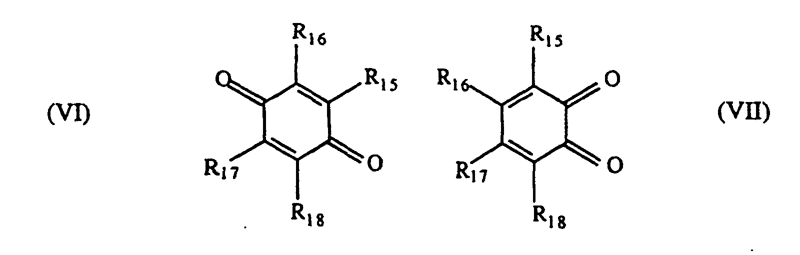 dans lesquelles :
R₁₅ désigne un atome d'hydrogène, d'halogène, un groupement sulfonique ou alcoxy,
R₁₆, R₁₇ et R₁₈, identiques ou différents désignent un atome d'hydrogène, d'halogène, un groupement hydroxy, alkyle, mono ou polyhydroxyalkyle, alkylhydroxyalkyle, alkylsulfonyle, carboxyalkyle, aminoalkyle, alkylaminoalkyle, (di-hydroxy)alkylaminoalkyle, ou alkyle-NR'R" (avec R' et R" désignant alkyle ou pouvant former ensemble avec l'atome d'azote auxquels ils sont rattachés un cycle aryle ou un hétérocycle à 5 ou 6 chaînons), un groupement aryle, un groupe amino pouvant être substitué par un alkyle ou un hydroxyalkyle,
R₁₅ et ₁₆, R₁₆ et R₁₇ ou R₁₇ et R₁₈ peuvent former conjointement avec les atomes auxquels ils sont rattachés un cycle aryle ou un hétérocycle à 5 ou 6 chaînons, substitué ou non;
ou aux sels cosmétiquement acceptables de ces composés.

6. Composition de teinture des fibres kératiniques, en particulier des fibres kératiniques humaines telles que les cheveux, **caractérisée par le fait qu'**elle comprend au moins une molécule comportant au moins une fonction amine, au moins une chaîne hydrocarbonée aliphatique et au moins un ammonium quaternaire et au moins un composé choisi parmi un aldéhyde, une cétone, une quinone, et un dérivé de la diiminoisoindoline ou de la 3-amino isoindolone dans un milieu approprié pour la teinture, permettant d'obtenir, sans agent oxydant, une teinture desdites fibres kératiniques.

7. Composition de teinture selon la revendication 6, **caractérisée par le fait que** la molécule comportant au moins une fonction amine, au moins une chaîne hydrocarbonée aliphatique et au moins un ammonium quaternaire est choisie parmi les composés définis selon la revendication 2.

8. Composition de teinture selon la revendication 6 ou 7, **caractérisée par le fait que** le composé choisi parmi un aldéhyde, une cétone, une quinone et un dérivé de la di-imino-isoindoline ou de la 3-amino-isoindoline est choisi parmi les composés définis Selon les revendications 3 à 5.

9. Composition de teinture selon l'une quelconque des revendications 6 à 8, **caractérisée par le fait qu'**elle a un pH compris entre 2 et 11.

10. Composition selon l'une quelconque des revendications 6 à 9, **caractérisée par le fait que** la molécule comportant au moins une fonction amine, au moins une chaîne hydrocarbonée aliphatique et au moins un ammonium quaternaire est présente dans une concentration allant de 0,1 à 10 % et de préférence de 0,5 à 5 % en poids par rapport au poids total de la composition.

11. Composition selon l'une quelconque des revendications 6 à 10, **caractérisée par le fait que** le composé choisi parmi un aldéhyde, une cétone, une quinone et un dérivé de la di-imino-isoindoline ou de la 3-amino-isoindolone est présent dans une concentration allant de 0,1 à 10 % et de préférence de 0,5 à 5 % en poids par rapport au poids total de la composition.

12. Composition selon l'une quelconque des revendications 6 à 11, **caractérisée par le fait que** le milieu approprié pour la teinture est un milieu aqueux constitué par de l'eau et/ou des solvants organiques choisis parmi les alcools, les glycols et les éthers de glycol, dans des proportions comprises entre 0,5 et 20% en poids par rapport au poids total de la composition.

13. Procédé de teinture des fibres kératiniques, en particulier des fibres kératiniques humaines telles que les cheveux, **caractérisé par le fait qu'**il consiste à appliquer sur lesdites fibres un composant (A) constitué d'une composition renfermant dans un milieu approprié pour la teinture, au moins une molécule comportant au moins une fonction amine au moins une chaîne hydrocarbonée aliphatique et au moins un ammonium quaternaire et au moins un composant (B) constitué d'une composition contenant dans un milieu approprié pour la teinture, au moins un composé choisi parmi un aldehyde, une cétone, une quinone et un dérivé de la di-imino-isoindoline ou de la 3-amino-isoindolone de façon à permettre le développement d'une teinture avec lesdites fibres kératiniques.

14. Procédé selon la revendication 13, **caractérisé par le fait que** la molécule comportant au moins une fonction amine au moins une chaîne hydrocarbonée aliphatique et au moins un ammonium quaternaire est choisie parmi les composés selon la revendication 2.

15. Procédé selon la revendication 13 ou 14, **caractérisé par le fait que** le composé choisi parmi un aldéhyde, une cétone, une quinone et un dérivé de la di-imino-isoindoline ou de la 3-amino-isoindolone est choisi parmi les composés selon l'une quelconque des revendications 3 à 5.

16. Procédé selon l'une quelconque des revendications 13 à 15, **caractérisé par le fait qu'**il consiste à mélanger les composants (A) et (B) juste avant emploi, à appliquer immédiatement là composition résultante sur les fibres kératiniques et à laisser agir pendant 1 à 60 minutes et préférentiellement pendant 1 à 30 minutes ; les fibres kératiniques étant ensuite rincées, lavées au shampooing, rincées à nouveau, puis séchées.

17. Procédé selon l'une quelconque des revendications 13 à 15, **caractérisé par le fait qu'**il consiste à appliquer sur les fibres kératiniques le composant (A), suivie ou précédée de l'application sur lesdites fibres du composant (B), à laisser agir chaque composant pendant 1 à 60 minutes et préférentiellement pendant 1 à 30 minutes, à procéder éventuellement au rinçage à l'eau entre chaque application; les fibres kératiniques étant ensuite rincées, lavées au shampooing, rincées à nouveau, puis séchées.

18. Agent de teinture des fibres kératiniques, et en particulier des fibres kératiniques humaines telles que les cheveux, **caractérisé par le fait qu'**il comporte les composants (A) et (B) tels que définis dans les revendications 13 à 17, sous forme séparée; les composants (A) et (B) étant destinés à être, soit mélangés tout juste avant emploi, soit appliqués de façon successive sur les fibres à traiter.

19. Dispositif à plusieurs compartiments ou "kit de teinture", **caractérisé par le fait qu'**il comporte au moins deux compartiments dont un renferme le composant (A) tel que défini dans la revendication 13 ou 14, et le second renferme le composant (B) tel que défini dans la revendication 13 ou 15.

20. Dispositif selon la revendication 19, **caractérisé par le fait que** le composant (A) et/ou le composant (B) se présente(nt) sous forme de composition anhydre et qu'il comporte un troisième compartiment contenant un milieu aqueux cosmétiquement acceptable approprié pour la teinture destiné à être mélangé avant emploi dans l'un ou les deux premiers compartiments renfermant chaque composant (A) ou (B).

## Patentansprüche

1. Verwendung mindestens eines Moleküls, das mindestens eine Aminofunktion, mindestens eine aliphatische Kohlenwasserstoffkette und mindestens eine quartäre Ammoniumgruppe aufweist, und mindestens einer Verbindung, die unter Aldehyden, Ketonen, Chinonen und Verbindungen der folgenden Formel (VIII) ausgewählt ist, zum Färben von Keratinfasern und insbesondere menschlichen Keratinfasern, wie Haaren: 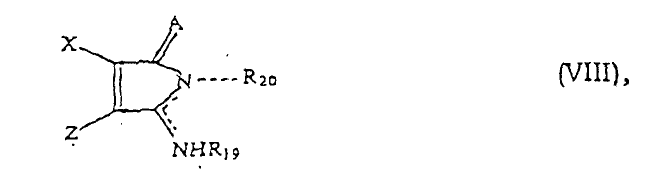 worin bedeuten:
die Gruppen R₁₉ und R₂₀, die gleich oder verschieden sind, ein Wasserstoffatom, Alkyl, Mono- oder Polyhydroxyalkyl, Alkylhydroxyalkyl, Aminoalkyl, Alkylaminoalkyl, (Dihydroxy)alkylaminoalkyl oder Alkyl-NR'R", worin R' und R" Alkyl bedeuten oder gemeinsam mit dem Stickstoffatom, an das sie gebunden sind, einen Arylring oder Heterocyclus mit 5 oder 6 Ringatomen bilden können,
A ein Sauerstoffatom oder NH,
X und Z gemeinsam einen Arylring oder Heterocyclus mit 5 oder 6 Kettengliedern, der substituiert oder unsubstituiert ist;
oder der kosmetisch akzeptablen Salze dieser Verbindungen, wodurch die Keratinfasern durch Reaktion ohne Oxidationsmittel gefärbt werden können.

2. Verwendung nach Anspruch 1, **dadurch gekennzeichnet, dass** das Molekül, das mindestens eine Aminofunktion, mindestens eine aliphatische Kohlenwasserstoffkette und mindestens eine quartäre Ammoniumgruppe enthält, unter den Verbindungen der folgenden Formel (I) ausgewählt ist: 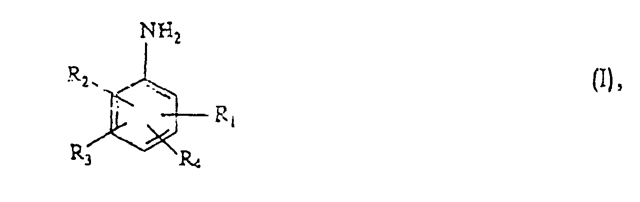 worin bedeuten:
• die Gruppen R₁, R₂, R₃, R₄, die gleich oder verschieden sind, ein Wasserstoffatom; ein Halogenatom; -NH₂, -OH, eine Gruppe Z; -COZ; -COOZ; Alkylcarbonyl; Aminoalkylcarbonyl; N-Alkylaminoalkylcarbonyl; N,N-Dialkylaminoalkylcarbonyl; Aminoalkylcarbonylalkyl; N-Alkylaminoalkylcarbonylalkyl; N,N-Dialkylaminoalkylcarbonylalkyl; Carboxy; Alkylcarboxy; Alkylsulfonyl; Aminosulfonyl; N-Alkylaminosulfonyl; N,N-Dialkylaminosulfonyl; Aminosulfonalkyl; N-Alkylaminosulfonylalkyl; N,N-Dialkylaminosulfonylalkyl; Carbamoyl; N-Alkylcarbamoyl; N,N-Dialkylcarbamoyl; Carbamoylalkyl; N-Alkylcarbamoylalkyl; N,N-Dialkylcarbamoylalkyl; Alkyl, Monohydroxyalkyl, Polyhydroxyalkyl, Alkoxyaryl, Trifluoralkyl, Cyano; ORᵢ; SRᵢ, ORᵢZ oder SRᵢZ oder eine Aminogruppe, die mit einer der folgenden Gruppen geschützt ist: Alkylcarboxy, Trifluoralkylcarbonyl, Aminoalkylcarbonyl, Carbonyl, N-Alkylaminoalkylcarbonyl, N,N-Dialkylaminoalkylcarbonyl, Alkylcarboxy, Carbamoyl, N-Alkylcarbamoyl, N,N-Dialkylcarbamoyl, Alkylsulfonyl, Aminosulfonyl, N-Alkylaminosulfonyl, N,N-Dialkylaminosulfonyl, Thiocarbamoyl, Formyl, -COZ oder -COOZ;
• Rᵢ Alkyl, Monohydroxyalkyl, Polyhydroxyalkyl, eine Gruppe Z, Alkoxyalkyl; Aryl; Benzyl, Carboxyalkyl, Alkylcarboxyalkyl, Cyanoalkyl, Carbamoylalkyl, N-Alkylcarbamoylalkyl; N,N-Dialkylcarbamoylalkyl; Trifluoralkyl; Aminosulfonylalkyl; N-Alkylaminosulfonylalkyl; N,N-Dialkylaminosulfonylalkyl; Alkylsulfinylalkyl; Alkylsulfonylalkyl; Alkylcarbonylalkyl; Aminoalkyl; eine Aminoalkylgruppe, bei der die Aminogruppe mit einer oder zwei gleichen oder verschiedenen Gruppen substituiert ist, die ausgewählt sind unter den Gruppen Alkyl, Monohydroxyalkyl; Polyhydroxyalkyl, Alkylcarbonyl, Formyl, Trifluoralkylcarbonyl, Alkylcarboxy, Carbamoyl, N-Alkylcarbamoyl, N,N-Dialkylcarbamoyl, Thiocarbamoyl, Alkylsulfonyl und unter den Gruppen Z, -COZ oder -COOZ;
• Z eine Gruppe der folgenden Formel (II): 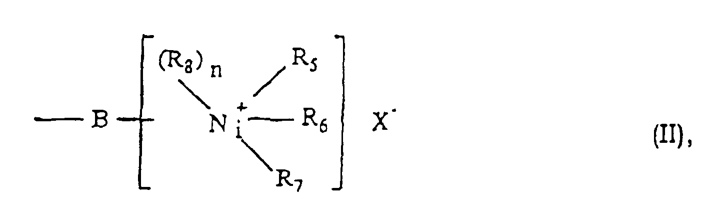 worin bedeuten:
• B eine geradkettige oder verzweigte Alkylkette, die durch ein oder mehrere Heteroatome, beispielsweise Sauerstoff, Schwefel oder Stickstoff, unterbrochen sein kann und die mit einer oder mehreren Hydroxygruppen oder C₁₋₆-Alkoxygruppen substituiert sein kann;
• R₅, R₆ und R₇, die identisch oder voneinander verschieden sind, Alkyl, Monohydroxyalkyl, Polyhydroxyalkyl, Alkoxyalkyl, Cyanoalkyl, Aryl, Benzyl, Carbamoylalkyl, Trialkylsilanalkyl oder eine Aminoalkylgruppe, deren Aminogruppe durch eine Gruppe Alkylcarbonyl, Carbamoyl oder Alkylsulfonyl geschützt ist; wobei zwei der Gruppen R₅, R₆ und R₇ gemeinsam mit dem Stickstoffatom, an das sie gebunden sind, einen 5- oder 6-gliedrigen Ring bilden können, der ein oder mehrere Heteroatome enthalten kann, wobei der Ring substituiert oder unsubstituiert vorliegen kann, wobei eine der Gruppen R₅, R₆ und R₇ auch eine Verbindungsgruppe B' einer zweiten Gruppe Z bedeuten kann, wobei B' die oben für die Gruppe B angegebenen Bedeutungen aufweist;
• X⁻ ein einwertiges oder zweiwertiges Anion, vorzugsweise ein Halogen, wie Chlor, Brom, Fluor oder Iod, Hydroxid, Hydrogensulfat oder ein Alkylsulfat, beispielsweise Methylsulfat oder Ethylsulfat;
• R₈ Alkyl, Monohydroxyalkyl, Polyhydroxyalkyl, Aryl; Benzyl; Aminoalkyl, eine Aminoalkylgruppe, deren Aminogruppe mit einer Gruppe Alkylcarbonyl, Carbamoyl oder Alkylsulfonyl geschützt ist; Carboxyalkyl; Cyanoalkyl; Carbamoylalkyl; Trifluoralkyl; Trialkylsilanalkyl; Sulfonamidoalkyl; Alkylcarboxyalkyl; Alkylsulfinylalkyl; Alkylsulfonylalkyl; Alkylketoalkyl; N-Alkylcarbamoylalkyl; N-Alkylsulfonamidoalkyl;
• n 0 oder 1, mit der Maßgabe, dass:
wenn n = 0, die Verbindungsgruppe B an das Stickstoffatom mit den Gruppen R₅ bis R₇ gebunden ist;
wenn n = 1, zwei der Gruppen R₅ bis R₇ gemeinsam mit dem Stickstoffatom, an das sie gebunden sind, einen gesättigten, 5- oder 6-gliedrigen Ring bilden, der ein oder mehrere Heteroatome enthalten kann, wobei der Ring substituiert oder unsubstituiert vorliegen kann, und die Verbindungsgruppe B von einem Kohlenstoffatom des gesättigten Rings und nicht von dem Stickstoffatom Nᵢ getragen wird; und
• die oben definierte Verbindung (I) mindestens eine Gruppe Z enthält;
und den kosmetisch akzeptablen Salzen dieser Verbindungen.

3. Verwendung nach Anspruch 1 oder 2, **dadurch gekennzeichnet, dass** der Aldehyd der folgenden Formel (III) entspricht: 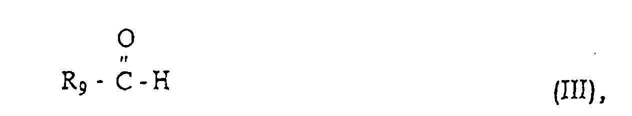 worin bedeuten:
R₉ eine Gruppe der folgenden Formel (III A): 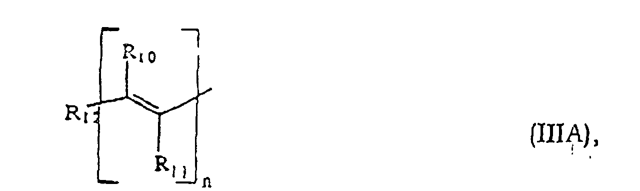 worin bedeuten:
die Gruppen R₁₀ und R₁₁, die gleich oder verschieden sind, ein Wasserstoffatom, Alkyl, Mono- oder Polyhydroxyalkyl, Alkylhydroxyalkyl, Alkoxy, -CF₃ oder -OCF₃,
wobei R₁₀ und R₁₁ auch gemeinsam mit den Atomen, an die sie gebunden sind, einen Arylring oder Heterocyclus mit 5 oder 6 Ringatomen bilden können, wobei die Ringe substituiert oder unsubstituiert vorliegen können;
n 0 oder eine ganze Zahl von 1 bis 3,
die Gruppe R₁₂ die für R₁₀ genannten Substituenten, eine Arylgruppe, eine substituierte oder unsubstituierte Alkylarylgruppe oder eine 5- oder 6-gliedrige, substituierte oder unsubstituierte heterocyclische Gruppe,
oder den kosmetisch akzeptablen Salzen dieser Verbindungen.

4. Verwendung nach einem der Ansprüche 1 bis 3, **dadurch gekennzeichnet, dass** das Keton den folgenden Formeln (IV) oder (V) entspricht: 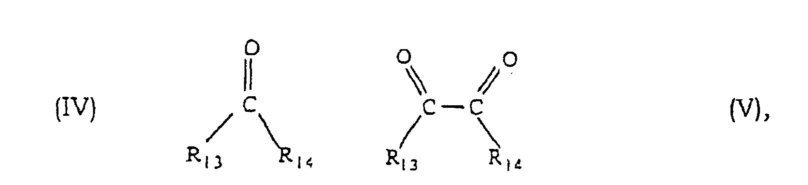 worin bedeuten:
R₁₃ die für die Gruppe R₉ genannten Substituenten,
R₁₄ Alkyl, Mono- oder Polyhydroxyalkyl, Alkylhydroxyalkyl, Aryl, Alkylaryl, eine substituierte oder unsubstituierte, 5- oder 6-gliedrige heterocyclische Gruppe,
wobei R₁₃ und R₁₄ auch gemeinsam mit den Atomen, an die sie gebunden sind, einen 5- oder 6-gliedrigen Arylring oder einen Heterocyclus, der Heteroatome wie N oder S enthält, bilden können, wobei der Ring selbst an einen 5- oder 6-gliedrigen Arylring oder einen Heterocyclus, der Heteroatome wie N oder S enthält, gebunden sein kann, wobei die Ringe substituiert oder unsubstituiert vorliegen können,
oder den kosmetisch akzeptablen Salzen dieser Verbindungen.

5. Verwendung nach einem der Ansprüche 1 bis 4, **dadurch gekennzeichnet, dass** das Chinon den folgenden Formeln (VI) und (VII) entspricht: 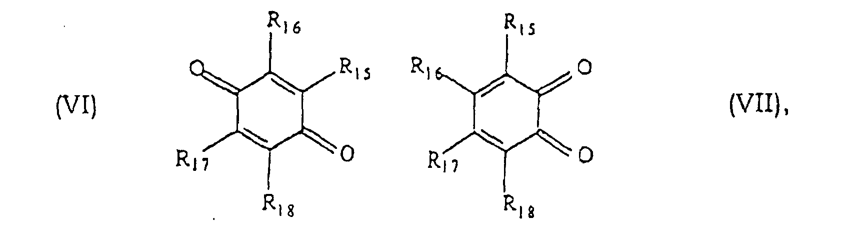 worin bedeuten:
R₁₅ ein Wasserstoffatom, ein Halogenatom, eine Sulfonsäuregruppe oder eine Alkoxygruppe,
die Gruppen R₁₆, R₁₇ und R₁₈, die gleich oder verschieden sind, ein Wasserstoffatom, ein Halogenatom, Hydroxy, Alkyl, Monooder Polyhydroxyalkyl, Alkylhydroxyalkyl, Alkylsulfonyl, Carboxyalkyl, Aminoalkyl, Alkylaminoalkyl, Dihydroxyalkylaminoalkyl oder Alkyl-NR'R" (wobei R' und R" Alkyl bedeuten oder gemeinsam mit dem Stickstoffatom, an das sie gebunden sind, einen Arylring oder einen Heterocylus mit 5 oder 6 Ringatomen bilden können), Aryl, eine Aminogruppe, die mit einer Alkylgruppe oder einer Hydroxyalkylgruppe substituiert sein kann,
wobei R₁₅ und R₁₆, R₁₆ und R₁₇ oder R₁₇ und R₁₈ gemeinsam mit den Atomen, an die sie gebunden sind, einen Arylring oder einen Heterocyclus mit 5 oder 6 Ringatomen, der substituiert oder unsubstituiert vorliegt, bilden können;
oder den kosmetisch akzeptablen Salzen dieser Verbindungen.

6. Zusammensetzung zum Färben von Keratinfasern und insbesondere menschlichen Keratinfasern, wie zum Färben der Haare, **dadurch gekennzeichnet, dass** sie mindestens ein Molekül, das mindestens eine Aminofunktion, mindestens eine aliphatische Kohlenwasserstoffkette und mindestens eine quartäre Ammoniumgruppe enthält, und mindestens eine Verbindung, die unter den Aldehyden, Ketonen, Chinonen und Diiminoisoindolinderivaten oder 3-Aminoisoindolonderivaten ausgewählt ist, in einem zum Färben geeigneten Medium enthält, wobei sie die Keratinfasern ohne Oxidationsmittel zu färben vermag.

7. Zusammensetzung zum Färben nach Anspruch 6, **dadurch gekennzeichnet, dass** das Molekül, das mindestens eine Aminofunktion, mindestens eine aliphatische Kohlenwasserstoffgruppe und mindestens eine quartäre Ammoniumgruppe enthält, unter den in Anspruch 2 definierten Verbindungen ausgewählt ist.

8. Zusammensetzung zum Färben nach Anspruch 6 oder 7, **dadurch gekennzeichnet, dass** die Verbindung, die unter den Aldehyden, Ketonen, Chinonen und Diiminoisoindolinderivaten oder 3-Aminoisoindolonderivaten ausgewählt ist, unter den in den Ansprüchen 3 bis 5 definierten Verbindungen ausgewählt ist.

9. Zusammensetzung zum Färben nach einem der Ansprüche 6 bis 8, **dadurch gekennzeichnet, dass** sie einen pH-Wert im Bereich von 2 bis 11 aufweist.

10. Zusammensetzung nach einem der Ansprüche 6 bis 9, **dadurch gekennzeichnet, dass** das Molekül, das mindestens eine Aminofunktion, mindestens eine aliphatische Kohlenwasserstoffkette und mindestens eine quartäre Ammoniumgruppe enthält, in einer Konzentration von 0,1 bis 10 % und vorzugsweise 0,5 bis 5 Gew.-%, bezogen auf das Gesamtgewicht der Zusammensetzung, enthalten ist.

11. , Zusammensetzung nach einem der Ansprüche 6 bis 10, **dadurch gekennzeichnet, dass** die Verbindung, die unter den Aldehyden, Ketonen, Chinonen und Diiminoisoindolinderivaten oder 3-Aminoisoindolonderivaten ausgewählt ist, in einer Konzentration von 0,1 bis 10 % und vorzugsweise 0,5 bis 5 Gew.-%, bezogen auf das Gesamtgewicht der Zusammensetzung, vorliegt.

12. Zusammensetzung nach einem der Ansprüche 6 bis 11, **dadurch gekennzeichnet, dass** das zum Färben geeignete Medium ein wässriges Medium ist, das aus Wasser und/oder organischen Lösungsmitteln besteht, die unter den Alkoholen, Glykolen und Glykolethern in Mengenanteilen von 0,5 bis 20 Gew.-%, bezogen auf das Gesamtgewicht der Zusammensetzung, ausgewählt sind.

13. Verfahren zum Färben von Keratinfasern und insbesondere menschlichen Keratinfasern, beispielsweise zum Färben der Haare, **dadurch gekennzeichnet, dass** es darin besteht, auf die Fasern eine Komponente (A), die aus einer Zusammensetzung besteht, die in einem zum Färben geeigneten Medium mindestens ein Molekül enthält, das mindestens eine Aminofunktion, mindestens eine aliphatische Kohlenwasserstoffkette und mindestens eine quartäre Ammoniumgruppe aufweist, und eine Komponente (B) aufzutragen, die aus einer Zusammensetzung besteht, die in einem zum Färben geeigneten Medium mindestens eine Verbindung enthält, die unter den Aldehyden, Ketonen, Chinonen und Diiminoisoindolinderivaten oder 3-Aminoisoindolonderivaten ausgewählt ist, um die Keratinfasern zu färben.

14. Verfahren nach Anspruch 13, **dadurch gekennzeichnet, dass** das Molekül, das mindestens eine Aminofunktion, mindestens eine aliphatische Kohlenwasserstoffkette und mindestens eine quartäre Ammoniumgruppe enthält, unter den Verbindungen gemäß Anspruch 2 ausgewählt ist.

15. Verfahren nach Anspruch 13 oder 14, **dadurch gekennzeichnet, dass** die Verbindung, die unter den Aldehyden, Ketonen, Chinonen und Diiminoisoindolinderivaten oder 3-Aminoisoindolonderivaten ausgewählt ist, unter den Verbindungen nach einem der Ansprüche 3 bis 5 ausgewählt ist.

16. Verfahren nach einem der Ansprüche 13 bis 15, **dadurch gekennzeichnet, dass** es darin besteht, die Komponenten (A) und (B) kurz vor der Anwendung zu vermischen, die resultierende Zusammensetzung sofort auf die Keratinfasern aufzutragen und 1 bis 60 Minuten und vorzugsweise 1 bis 30 Minuten einwirken zu lassen, wobei die Keratinfasern dann gespült, mit Haarwaschmittel gewaschen, nochmals gespült und anschließend getrocknet werden.

17. Verfahren nach einem der Ansprüche 13 bis 15, **dadurch gekennzeichnet, dass** es darin besteht, die Komponente (A) auf die Keratinfasern aufzutragen und davor oder danach die Komponente (B) auf die Fasern aufzubringen, jede Komponente 1 bis 60 Minuten und vorzugsweise 1 bis 30 Minuten einwirken zu lassen und gegebenenfalls zwischen den Anwendungen mit Wasser zu spülen, wobei die Keratinfasern dann gespült, mit Haarwaschmittel gewaschen, nochmals gespült und anschließend getrocknet werden.

18. Mittel zum Färben von Keratinfasern und insbesondere menschlichen Keratinfasern, wie zum Färben der Haare, **dadurch gekennzeichnet, dass** es die Komponenten (A) und (B) nach den Ansprüchen 13 bis 17 in getrennter Form enthält, wobei die Komponenten (A) und (B) dazu vorgesehen sind, entweder kurz vor der Anwendung vermischt zu werden oder nacheinander auf die zu behandelnden Fasern aufgetragen zu werden.

19. Vorrichtung mit mehreren Abteilungen oder "Kit zum Färben", **dadurch gekennzeichnet, dass** sie mindestens zwei Abteilungen enthält, wobei eine Abteilung die in Anspruch 13 oder 14 definierte Komponente (A) enthält und eine zweite Abteilung die in den Ansprüchen 13 oder 15 definierte Komponente (B) enthält.

20. Vorrichtung nach Anspruch 19, **dadurch gekennzeichnet, dass** die Komponente (A) und/oder die Komponente (B) in Form einer wasserfreien Zusammensetzung vorliegt und dadurch, dass sie eine dritte Abteilung enthält, die ein zum Färben geeignetes, kosmetisch akzeptables, wässriges Medium enthält, das vor der Anwendung in einer oder beiden Abteilungen, die jeweils die Komponente (A) oder (B) enthalten, vermischt werden soll.

## Claims

1. Use, for dyeing keratin fibres, in particular human keratin fibres such as human hair, of at least one molecule comprising at least one amine functional group, at least one aliphatic hydrocarbon-based chain and at least one quaternary ammonium and of at least one compound chosen from an aldehyde, a ketone, a quinone and a compound of formula (VIII): 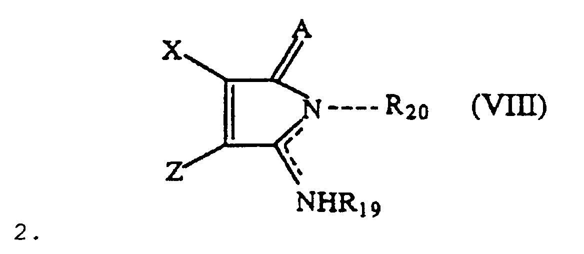 in which:
R₁₉ and R₂₀, which may be identical or different, denote a hydrogen atom, an alkyl, mono- or polyhydroxyalkyl, alkylhydroxyalkyl, aminoalkyl, alkylaminoalkyl or (dihydroxy)alkylaminoalkyl group or an alkyl-NR'R" group, with R' and R" denoting alkyl or possibly forming, together with the nitrogen atom to which they are attached, an aryl ring or a 5- or 6-membered heterocycle),
A denotes an oxygen atom or NH,
X and Z together form a substituted or unsubstituted aryl ring or a 5- or 6-membered heterocycle; or to the cosmetically acceptable salts of these compounds,
in order to obtain, by reaction without an oxidizing agent, a coloration of the said keratin fibres.

2. Use according to Claim 1, **characterized in that** the molecule comprising at least one amine functional group, at least one aliphatic hydrocarbon-based chain and at least one quaternary ammonium is chosen from the compounds of formula (I) below: 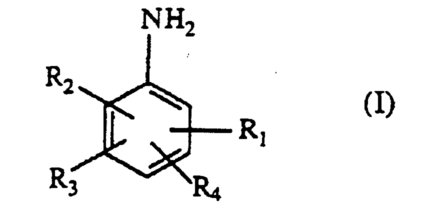 in which:
• R₁, R₂, R₃ and R₄, which may be identical or different, represent a hydrogen atom; a halogen atom, an -NH₂ group, an -OH group; a group Z; a group -COZ; a group -COOZ; an alkylcarbonyl radical; an aminoalkylcarbonyl radical; an N-alkylaminoalkylcarbonyl radical; an N,N-dialkylaminoalkylcarbonyl radical; an aminoalkylcarbonylalkyl radical; an N-alkylaminoalkylcarbonylalkyl radical; an N,N-dialkylaminoalkylcarbonylalkyl radical; a carboxyl radical; an alkylcarboxyl radical; an alkylsulphonyl radical; an aminosulphonyl radical; an N-alkylaminosulphonyl radical; an N,N-dialkylaminosulphonyl radical; an aminosulphonylalkyl radical; an N-alkylaminosulphonylalkyl radical; an N,N-dialkylaminosulphonylalkyl radical; a carbamyl radical; an N-alkylcarbamyl radical; an N,N-dialkylcarbamyl radical; a carbamylalkyl radical; an N-alkylcarbamylalkyl radical; an N,N-dialkylcarbamylalkyl radical; an alkyl, monohydroxyalkyl, polyhydroxyalkyl, alkoxyalkyl or trifluoroalkyl radical or a cyano radical; a group ORᵢ, SRᵢ, ORᵢZ or SRᵢZ or an amino group protected with an alkylcarboxyl, trifluoroalkylcarbonyl, aminoalkylcarbonyl, carbonyl, N-alkylaminoalkylcarbonyl, N,N-dialkylaminoalkylcarbonyl, alkylcarboxyl, carbamyl, N-alkylcarbamyl, N,N-dialkylcarbamyl, alkylsulphonyl, aminosulphonyl, N-alkylaminosulphonyl, N,N-dialkylaminosulphonyl, thiocarbamyl or formyl radical, a group -COZ or a group -COOZ;
• Rᵢ denotes an alkyl, monohydroxyalkyl or polyhydroxyalkyl radical, a group Z, an alkoxyalkyl radical; an aryl radical; a benzyl radical, a carboxyalkyl radical, an alkylcarboxyalkyl radical, a cyanoalkyl radical, a carbamylalkyl radical or an N-alkylcarbamylalkyl radical; an N,N-dialkylcarbamylalkyl radical; a trifluoroalkyl radical; an aminosulphonylalkyl radical; an N-alkylaminosulphonylalkyl radical; an N,N-dialkylaminosulphonylalkyl radical; an alkylsulphinylalkyl radical; an alkylsulphonylalkyl radical; an alkylcarbonylalkyl radical; an aminoalkyl radical; an aminoalkyl radical in which the amine is substituted with one or two radicals, which may be identical or different, chosen from alkyl, monohydroxyalkyl, polyhydroxyalkyl, alkylcarbonyl, formyl, trifluoroalkylcarbonyl, alkylcarboxyl, carbamyl, N-alkylcarbamyl, N,N-dialkylcarbamyl, thiocarbamyl and alkylsulphonyl radicals and from the groups Z, -COZ or -COOZ;
Z representing a group of formula (II) below: 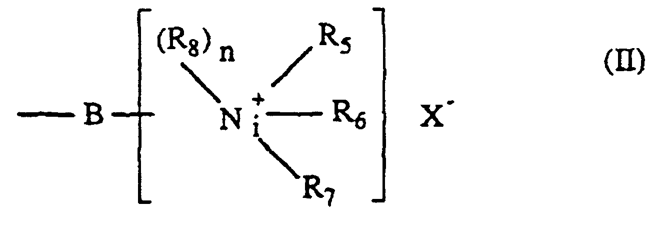 in which:
• B represents a linear or branched alkyl chain, which may be interrupted by one or more hetero atoms such as oxygen, sulphur or nitrogen atoms, and which may be substituted with one or more hydroxyl or C₁-C₆ alkoxy radicals;
• R₅, R₆ and R₇, which may be identical or different, represent an alkyl radical, a monohydroxyalkyl radical, a polyhydroxyalkyl radical, an alkoxyalkyl radical, a cyanoalkyl radical, an aryl radical, a benzyl radical, a carbamylalkyl radical, a trialkylsilanealkyl radical or an aminoalkyl radical in which the amine is protected with an alkylcarbonyl, carbamyl or alkylsulphonyl radical; two of the radicals R₅, R₆ and R₇ can also form, together with the nitrogen atom to which they are attached, a 5- or 6-membered ring which can contain one or more hetero atoms, the said ring possibly being substituted or unsubstituted,
one of the radicals R₅, R₆ and R₇ can also represent a linker arm B' of a second radical Z, B' having the same meaning as that indicated above for the radical B;
• X⁻ represents a monovalent or divalent anion, and preferably represents a halogen atom such as chlorine, bromine, fluorine or iodine, a hydroxide, a hydrogen sulphate or an alkyl sulphate such as, for example, a methyl sulphate or an ethyl sulphate;
• R₈ represents an alkyl, monohydroxyalkyl or polyhydroxyalkyl radical, an aryl radical; a benzyl radical; an aminoalkyl radical, an aminoalkyl radical in which the amine is protected with an alkylcarbonyl, carbamyl or alkylsulphonyl radical; a carboxyalkyl radical; a cyanoalkyl radical; a carbamylalkyl radical; a trifluoroalkyl radical; a trialkylsilanealkyl radical; a sulphonamidoalkyl radical; an alkylcarboxyalkyl radical; an alkylsulphinylalkyl radical; an alkylsulphonylalkyl radical; an alkylketoalkyl radical; an N-alkylcarbamylalkyl radical; an N-alkylsulphonamidoalkyl radical;
• n is an integer equal to 0 or 1, it being understood that:
when n = 0, the linker arm B is attached to the nitrogen atom bearing the radicals R₅ to R₇;
when n = 1, then two of the radicals R₅ to R₇ form, together with the nitrogen atom to which they are attached, a saturated 5- or 6-membered ring which can contain one or more hetero atoms, the said ring possibly being substituted or unsubstituted, and the linker arm B is borne by a carbon atom of the said saturated ring outside of the nitrogen atom Nᵢ; and
• the compound (I) defined above contains at least one group Z;
and the cosmetically acceptable salts of these compounds.

3. Use according to Claim 1 or 2, **characterized in that** the aldehyde corresponds to formula (III) below: 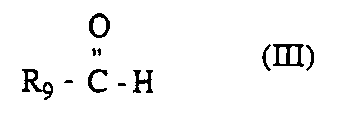 in which:
R₉ denotes a group of formula (III A) below: 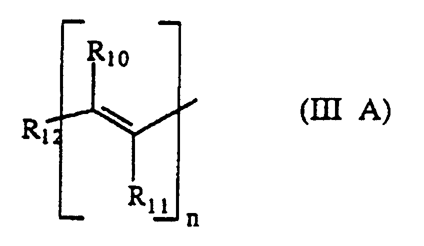 in which:
R₁₀ and R₁₁, which may be identical or different, denote a hydrogen atom or an alkyl, mono- or polyhydroxyalkyl, alkylhydroxyalkyl, alkoxy, -CF₃ or -OCF₃ group,
R₁₀ and R₁₁ can also form, together with the atoms to which they are attached, an aryl ring or a 5- or 6-membered heterocyclic ring, it being possible for the said rings to be substituted or unsubstituted;
n denotes an integer from 0 to 3,
R₁₂ denotes the substituents denoted by R₁₀, a substituted or unsubstituted aryl or alkylaryl group or a substituted or unsubstituted 5- or 6-membered heterocyclic group,
or to the cosmetically acceptable salts of these compounds.

4. Use according to any one of Claims 1 to 3, **characterized in that** the ketone corresponds to formula (IV) or (V) below: 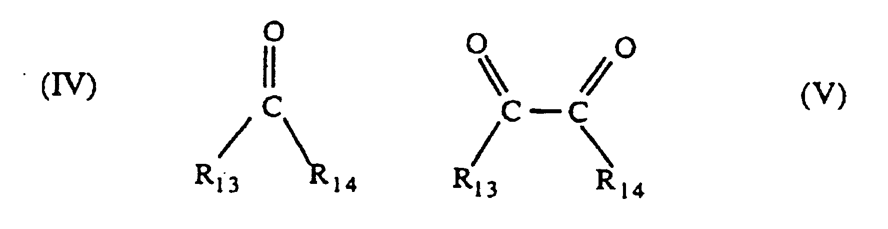 in which:
R₁₃ denotes the substituents denoted by R₉,
R₁₄ denotes an alkyl, mono- or polyhydroxyalkyl or alkylhydroxyalkyl group, or a substituted or unsubstituted aryl, alkylaryl or 5- or 6-membered heterocyclic group,
R₁₃ and R₁₄ can also form, together with the atoms to which they are attached, a 5- or 6-membered aryl ring or a heterocyclic ring comprising hetero atoms such as N or S, it being possible for the said ring itself to be attached to a 5- or 6-membered aryl ring or to a heterocycle comprising hetero atoms such as N or S, it being possible for the said rings to be substituted or unsubstituted,
or to the cosmetically acceptable salts of these compounds.

5. Use according to any one of Claims 1 to 4, **characterized in that** the quinone corresponds to formulae (VI) and (VII) below: 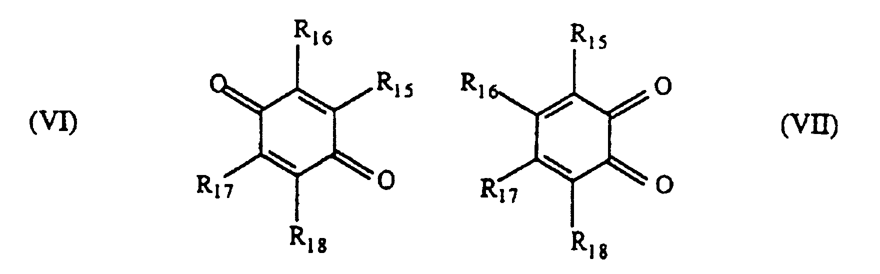 in which:
R₁₅ denotes a hydrogen or halogen atom or a sulphonic or alkoxy group,
R₁₆, R₁₇ and R₁₈, which may be identical or different, denote a hydrogen or halogen atom, a hydroxyl, alkyl, mono- or polyhydroxyalkyl, alkylhydroxyalkyl, alkylsulphonyl, carboxyalkyl, aminoalkyl, alkylaminoalkyl, (dihydroxy)alkylaminoalkyl or alkyl-NR'R" group (with R' and R" denoting alkyl or possibly forming, together with the nitrogen atom to which they are attached, an aryl ring or a 5- or 6-membered heterocycle), an aryl group or an amino group which can be substituted with an alkyl or a hydroxyalkyl,
R₁₅ and R₁₆, R₁₆ and R₁₇ or R₁₇ and R₁₈ can form, together with the atoms to which they are attached, a substituted or unsubstituted aryl ring or 5- or 6-membered heterocycle;
or to the cosmetically acceptable salts of these compounds.

6. Composition for dyeing keratin fibres, in particular human keratin fibres such as the hair, **characterized in that** it comprises at least one molecule comprising at least one amine functional group, at least one aliphatic hydrocarbon-based chain and at least one quaternary ammonium and at least one compound chosen from an aldehyde, a ketone, a quinone and a diiminoisoindoline or 3-aminoisoindolone derivative in a medium which is suitable for dyeing, in order to obtain, without an oxidizing agent, a coloration of the said keratin fibres.

7. Dye composition according to Claim 6, **characterized in that** the molecule comprising at least one amine functional group, at least one aliphatic hydrocarbon-based chain and at least one quaternary ammonium is chosen from the compounds defined according to Claim 2.

8. Dye composition according to Claim 6 or 7, **characterized in that** the compound chosen from an aldehyde, a ketone, a quinone and a diiminoisoindoline or 3-aminoisoindolone derivative is chosen from the compounds defined according to Claims 3 to 5.

9. Dye composition according to any one of Claims 6 to 8, **characterized in that** it has a pH of between 2 and 11.

10. Composition according to any one of Claims 6 to 9, **characterized in that** the molecule comprising at least one amine functional group, at least one aliphatic hydrocarbon-based chain and at least one quaternary ammonium is present in a concentration ranging from 0.1% to 10% and preferably from 0.5% to 5% by weight relative to the total weight of the composition.

11. Composition according to any one of Claims 6 to 10, **characterized in that** the compound chosen from an aldehyde, a ketone, a quinone and a diiminoisoindoline or 3-aminoisoindolone derivative is present in a concentration ranging from 0.1% to 10% and preferably from 0.5% to 5% by weight relative to the total weight of the composition.

12. Composition according to any one of Claims 6 to 11, **characterized in that** the medium which is suitable for dyeing is an aqueous medium consisting of water and/or organic solvents chosen from alcohols, glycols and glycol ethers, in proportions of between 0.5% and 20% by weight relative to the total weight of the composition.

13. Process for dyeing keratin fibres, in particular human keratin fibres such as the hair, **characterized in that** it consists in applying a component (A) consisting of a composition containing, in a medium which is suitable for dyeing, at least one molecule comprising at least one amine functional group, at least one aliphatic hydrocarbon-based chain and at least one quaternary ammonium, and at least one component (B) consisting of a composition containing, in a medium which is suitable for dyeing, at least one compound chosen from an aldehyde, a ketone, a quinone and a di-iminoisoindoline or 3-aminoisoindolone derivative, to the said fibres so as to allow the development of a coloration on the said keratin fibres.

14. Process according to Claim 13, **characterized in that** the molecule comprising at least one amine functional group, at least one aliphatic hydrocarbon-based chain and at least one quaternary ammonium is chosen from the compounds according to Claim 2.

15. Process according to Claim 13 or 14, **characterized in that** the compound chosen from an aldehyde, a ketone, a quinone and a diiminoisoindoline or 3-aminoisoindolone derivative is chosen from the compounds according to any one of Claims 3 to 5.

16. Process according to any one of Claims 13 to 15, **characterized in that** it consists in mixing components (A) and (B) just before use, in immediately applying the resulting composition to the keratin fibres and in leaving the composition to act for 1 to 60 minutes and preferably for 1 to 30 minutes; the keratin fibres then being rinsed, washed with shampoo, rinsed again and then dried.

17. Process according to any one of Claims 13 to 15, **characterized in that** it consists in applying component (A) to the keratin fibres, followed or preceded by application of component (B) to the said fibres, in leaving each component to act for 1 to 60 minutes and preferably for 1 to 30 minutes, and optionally in rinsing with water between each application; the keratin fibres then being rinsed, washed with shampoo, rinsed again and then dried.

18. Agent for dyeing keratin fibres, and in particular human keratin fibres such as the hair, **characterized in that** it separately comprises components (A) and (B) as defined in Claims 13 to 17; the components (A) and (B) being intended to be either mixed immediately before use or applied successively to the fibres to be treated.

19. Multi-compartment device or "dyeing kit", **characterized in that** it comprises at least two compartments, one of which contains component (A) as defined in Claim 13 or 14, and the second of which contains component (B) as defined in Claim 13 or 15.

20. Device according to Claim 19, **characterized in that** component A and/or component B is (are) in the form of an anhydrous composition, and **in that** it comprises a third compartment containing a cosmetically acceptable aqueous medium which is suitable for dyeing and which is intended to be mixed, before use, into one or both of the first compartments containing each component (A) or (B).
